# NEUE EUROPÄISCHE PATENTSCHRIFT

(11) **EP 1 633 468 B2**
(45) Veröffentlichungstag und Bekanntmachung des Hinweises auf die Entscheidung über den Einspruch: **19.10.2016**
(45) Hinweis auf die Patenterteilung: 14.01.2009
(21) Anmeldenummer: 04736308.0
(22) Anmeldetag: 08.06.2004
(51) Int. Cl.: B01J 13/02, B01J 13/04, C11D 3/50

(54) **FLÜSSIGE WASCH- UND REINIGUNGSMITTELZUSAMMENSETZUNGEN ENTHALTEND LAGERSTABILE KAPSELN AUF BASIS VON PEROXYCARBONSÄUREN**
LIQUID WASHING OR CLEANING AGENT COMPOSITIONS COMPRISING PERCARBOXYLIC ACID-BASED CAPSULES HAVING A LONG SHELF LIFE
COMPOSITIONS LIQUIDES D'AGENT DE LAVAGE OU DE NETTOYAGE COMPRENANT DES CAPSULES A BASE D'ACIDES PEROXYCARBONIQUES PRESENTANT UNE BONNE STABILITE AU STOCKAGE

(30) Priorität: 13.06.2003 DE 10327127; 22.12.2003 DE 10361100
(43) Veröffentlichungstag der Anmeldung: 15.03.2006
(73) Patentinhaber: Henkel AG & Co. KGaA, 40589 Düsseldorf (DE)
(72) Erfinder: SCHMIEDEL, Peter, 40599 Düsseldorf (DE); KAISER, Heribert, 40223 Düsseldorf (DE); SCHOLL, Elke, 40489 Düsseldorf (DE); VON RYBINSKI, Wolfgang, 40593 Düsseldorf (DE)
(86) Internationale Anmeldenummer: PCT/EP2004/006168
(87) Internationale Veröffentlichungsnummer: WO 2004/110612

(56) Entgegenhaltungen:
- EP-A- 0 272 402
- EP-A- 0 435 379
- WO-A1-00/27979
- CH-A- 363 329
- GB-A- 1 156 240
- GB-A- 2 032 421
- US-A- 3 494 786
- US-A- 3 494 786
- US-A- 3 936 537
- US-A- 4 170 453
- US-A- 5 049 298
- US-A- 5 296 156
- US-A- 5 478 488
- US-A- 5 536 435
- US-A- 5 707 953
- US-A- 5 770 551
- US-A- 6 066 365

## Beschreibung

Die vorliegende Erfindung betrifft flüssige Wasch- und Reinigungsmittel.

Bei flüssigen, insbesondere wasserhaltigen Wasch- und Reinigungsmitteln, die sich aufgrund ihrer positiven Produkteigenschaften, wie einer besseren und schnelleren Löslichkeit sowie Anwendbarkeit, einer zunehmenden Beliebtheit erfreuen ist die Einformulierung bzw. Inkorporierung von Bleich(mittel)komponenten aus zahlreichen Gründen problematisch. So verlieren eingesetzte Bleichmittel beispielsweise aufgrund von Zersetzungs- bzw. Hydrolysereaktionen und Inkompatibilitäten gegenüber anderen Bestandteilen der Waschmittelformulierung, wie zum Beispiel Enzymen oder Tensiden, häufig schon bei der Lagerung ihre Aktivität.

Als nachteilige Folgen verliert die Waschmittelformulierung hierdurch deutlich an Waschleistung, insbesondere Bleichvermögen, so daß insbesondere bleichbare Verschmutzungen nicht mehr zufriedenstellend entfernt werden können.

Die üblicherweise für feste Waschmittelformulierungen verwendeten Bleichmittelkomponenten, wie beispielsweise Perborate oder Percarbonate, sind äußerst feuchtigkeitsempfindlich, so daß in einem flüssigen und insbesondere wasserhaltigen Wasch- und Reinigungsmittel aufgrund des Verlustes von Aktivsauerstoff häufig innerhalb weniger Tage ein deutlicher Rückgang ihrer Bleichwirkung zu beobachten ist. Daher können derartige Wirkstoffe zum Zeitpunkt ihrer Anwendung, insbesondere in der Waschflotte, häufig ihre Bleichwirkung bereits verloren haben und somit unwirksam sein.

Demgegenüber sind Peroxycarbonsäuren, insbesondere Imidoperoxycarbonsäuren, deren wichtigster Vertreter Phthalimidocapronsäure (PAP) ist, effizienter und weniger hydrolyseempfindlich und im Stand der Technik als Bleichmittel für Wasch- und Reinigungsmittel bekannt. Jedoch ist ihre Lagerstabilität bei weitem nicht ausreichend, um eine langfristige Einsetzbarkeit des entsprechenden Wasch- oder Reinigungsmittel ohne einhergehenden Aktivitätsverlust zu gewährleisten. Besonders problematisch ist der Einsatz von Peroxycarbonsäuren, insbesondere Imidoperoxycarbonsäuren, in flüssigen Wasch- und Reinigungsmitteln.

Aufgrund der Nachteile, die sich in bezug auf eine Veränderung der Wasch- oder Reinigungsmittelformulierung infolge des Abbaus von Imidoperoxycarbonsäuren, insbesondere PAP, ergeben, wurde im Stand der Technik versucht, die Imidoperoxycarbonsäuren (z. B. PAP) wirkungsvoll zu verkapseln, so daß die Imidoperoxycarbonsäuren mit ihrer Umgebung, insbesondere den übrigen Komponenten der Wasch- bzw. Reinigungsmittelformulierung, nicht in direkten Kontakt geraten können bzw. der Kontakt der Imidoperoxycarbonsäuren mit ihrer Umgebung reduziert wird.

So werden im Stand der Technik häufig Wachse als Schutzhülle für empfindliche Waschmittelzusatzstoffe, wie beispielsweise Peroxycarbonsäuren, verwendet. In diesem Zusammenhang beschreibt die EP 0 510 761 B1 bzw. die zu derselben Patentfamilie gehörende US-A-5 230 822 ein allgemeines Verfahren zur Verkapselung von Waschmittetzusatzstoffen beliebiger Art, wie z. B. Enzymen, Bleichmitteln, unter anderem auch PAP, Bleichmittelvorläufern und Bleichkatalysatoren. Die dort beschriebene Verwendung eines Wachses, dessen Schmelzpunkt zwischen 40 °C und 50 °C liegt, führt dazu, daß die Wirksubstanzen in der Waschflotte erst bei Temperaturen oberhalb des Schmelzpunktes des verwendeten Wachses freigesetzt werden, was vor dem Hintergrund der Entwicklung leistungsfähiger Wasch- und Reinigungsmittelformulierungen und der Einsparung von Energiekosten nachteilig ist, da insbesondere bei etwa 30 °C gewaschen werden soll. Weiterhin weist die Verwendung eines Wachses mit einem hohen Schmelzpunkt den Nachteil auf, daß es insbesondere bei niedrigen Temperaturen Rückstände auf der Wäsche verursacht, da es bei diesen Temperaturen nicht vollständig emulgiert wird.

Weiterhin beschreibt die WO 93/045545 A1 wirkstoffhaltige Kapseln, die unter Verwendung einer Trägerphase und eines Blockcopolymers mit Hilfe eines Miniemulsionsverfahrens hergestellt werden. Die dort angeführten Wirkstoffe müssen in der Ölphase gelöst sein, so daß die dort beschriebenen Kapselsysteme weitgehend auf in der Ölphase lösliche, insbesondere hydrophobe Aktiv- bzw. Wirkstoffe beschränkt sind. Bleichmittel sind dort nicht erwähnt. Zur Herstellung der Miniemulsion ist eine Hochdruckhomogenisation erforderlich.

Darüber hinaus wurde im Stand der Technik versucht, andere Methoden zur Stabilisierung von Peroxycarbonsäuren zu entwickeln, wobei ein Schwerpunkt auf Modifizierungen der flüssigen Formulierungen gelegt wurde, in denen die Peroxycarbonsäuren eingearbeitet werden kann.

So beschreibt die EP 0 334 405 B1 eine wäßrige Bleichmittelzusammensetzung, welche eine teilchenförmige und im wesentlichen wasserunlösliche organische Peroxycarbonäure und zudem ein Alkansulfonat und eine Fettsäure enthält. Dabei soll eine stabile Dispersion der Peroxycarbonsäure erhalten werden und insbesondere eine Phasenseparierung der Peroxycarbonsäure von dem wäßrigen Medium verhindert werden. Ein Schutz im Sinne einer chemischen bzw. physikalischen Stabilisierung der Peroxycarbonsäure durch das Aufbringen einer Verkapselung auf die Peroxycarbonsäure ist nicht vorgesehen.

In diesem Zusammenhang beschreibt auch die EP 0 334 404 B1 eine Stabilisierung der Peroxycarbonsäure gegen Phasentrennung in einer wäßrigen Bleichmittelzusammensetzung durch Zugabe einer Fettsäure. Wie in der EP 0 334 405 B1, so ist auch in der EP 0 334 404 B1 eine Verkapselung der Peroxycarbonsäure in diesem Dokument nicht vorgesehen.

Die EP 0 337 516 A2 beschreibt wäßrige Bleichmittelzusammensetzungen auf Basis einer wasserunlöslichen organischen Peroxycarbonsäure. Die Stabilität der Peroxycarbonsäure soll durch das Vorhandensein von Alkansulfonaten und Natriumsulfat in der flüssigen Zusammensetzung erhöht werden. Auch hier fehlt jeglicher Hinweis auf eine Verkapselung.

Weiterhin beschreibt die WO 94/13776 wäßrige Bleichmittelzusammensetzungen auf Basis von Peroxycarbonsäuren, wobei im allgemeinen eine Stabilisierung der Peroxycarbonsäure durch Anpassungen der flüssigen Zusammensetzungen - also dem Dispersionsmittel - vorgesehen sind. Eine weitere Stabilisierung der Peroxycarbonsäure, insbesondere durch eine Verkapselung bzw. Hüllstruktur, die auf die Peroxycarbonsäure aufgebracht wird, ist in dieser Schrift ebenfalls nicht genannt.

In der EP 0 442 549 B1 wird die Zugabe von Wasserstoffperoxid zu einer flüssigen Bleichmittelzusammensetzung beschrieben, welche eine teilchenförmige und im wesentlichen wasserunlösliche organische Peroxycarbonsäure, beispielsweise PAP, umfaßt. Hierdurch soll die Bleichleistung der Peroxycarbonsäure insbesondere bei höheren Temperaturen verbessert werden. Eine Stabilisierung der Peroxycarbonsäure findet auch hier über Einstellung bzw. Anpassung der flüssigen Matrix statt.

Schließlich ist in der EP 0 435 379 A2 eine Beschichtung von Imidoperoxycarbonsäuren mit Fettsäuren, Fettalkoholen bzw. Fettestern sowie eine Stabilisierung einer die Imidoperoxycarbonsäure enthaltenden Dispersion durch Zugabe eines Alkalimetallsalzes einer Alkylbenzolsulfonsäure bzw. eines anorganischen Satzes zu dieser Dispersion beschrieben. Bei der dort geschilderten Beschichtung handelt es sich um ein Wachs bzw. wachsähnliche Substanzen, die erst bei höheren Temperaturen aufgelöst werden und bei der Anwendung zu unerwünschten Rückständen führen können.

Vor diesem Hintergrund besteht somit eine Aufgabe der vorliegenden Erfindung darin; eine Einlagerung oder Verkapselung bzw. Beschichtung von Peroxycarbonsäuren, insbesondere Imidoperoxycarbonsäuren, wie Phthalimidoperoxycapronsäure (PAP), mit gegenüber dem Stand der Technik verbesserten Eigenschaften sowie ein entsprechendes Herstellungsverfahren bereitzustellen.

Eine weitere Aufgabe der vorliegenden Erfindung besteht darin, Peroxycarbonsäuren, welche Imidoperoxycarbonsäuren sind wie Phthalimidoperoxycapronsäure (PAP), in lagerstabiler Form bereitzustellen. Dabei soll eine Konfektionierungsform von Peroxycarbonsäuren entwickelt werden, die ein Auf- oder Anlösen der Peroxycarbonsäuren im Zustand konzentrierter Dispersionen zumindest im wesentlichen verhindert oder verringert und vorzugsweise deren festen bzw. kristallinen Zustand auch in Gegenwart von Tensiden oder einer sonstigen Umgebung, die ein Lösungsvermögen für Peroxycarbonsäuren besitzt, insbesondere in Wasch- oder Reinigungsmittelformulierungen, erhält. In diesem Zusammenhang soll insbesondere ein Kontakt der Peroxycarbonsäuren mit der Umgebung zumindest im wesentlichen verhindert bzw. zumindest verringert werden.

Noch eine weitere Aufgabe der vorliegenden Erfindung ist die Bereitstellung von Kapseln, die mit Peroxycarbonsäuren in fester Form beladen sind und zu einer guten Stabilisierung der Peroxycarbonsäuren und damit zu einer verbesserten Lagerstabilität führen. Dabei sollen im Rahmen der vorliegenden Erfindung Kapseln bereitgestellt werden, die sich während des Waschvorgangs schnell auflösen und die Freisetzung der Peroxycarbonsäure, insbesondere in die Waschflotte, nicht beeinträchtigen. Insbesondere sollen im Rahmen der vorliegenden Erfindung Kapseln bereitgestellt werden, die während des Waschvorgangs keine Rückstände hinterlassen, was durch ein im wesentlichen vollständiges Auflösen und/oder Solubilisieren bzw. Dispergieren der Kapseln erreicht werden soll. Dabei soll das Verfahren zur Herstellung dieser Kapseln gleichermaßen kostengünstig, technisch einfach und im industriellen Maßstab anwendbar sein.

Die Anmelderin hat nun überraschenderweise herausgefunden, daß organische Peroxycarbonsäuren, nämlich Imidoperoxycarbonsäuren (z. B. PAP), durch eine Verkapselung mit einem anorganischen Salz im hohen Maße - bei gleichzeitiger guter Anwendbarkeit - stabilisiert werden können.

Gegenstand der vorliegenden Erfindung sind flüssige Wasch- und Reinigungsmittelzusammensetzungen, enthaltend Dispersionen, insbesondere wäßrige Dispersionen, enthaltend mit mindestens einer organischen Peroxycarbonsäure, welche eine Imidoperoxycarbonsäure, vorzugsweise PAP, ist, beladene Kapseln, umfassend mindestens eine Imidoperoxycarbonsäure, vorzugsweise PAP, welche zumindest im wesentlichen mit einer Kapselhülle auf Basis eines anorganischen Salzes umhüllt/und/oder beschichtet sind, wobei die Kapseln eine Kapselhülle auf Basis mindestens eines anorganischen Salzes und einen Kapselkern auf Basis mindestens einer Imidoperoxycarbonsäure umfassen, und ein anorganisches Salz, welches die Löslichkeit des die Kapselhülle bildenden anorganischen Salzes in der Dispersion herabsetzt. Dabei wird im Rahmen der vorliegenden Erfindung die zu verkapselnde Peroxycarbonsäure in einer Kapselhülle eingelagert bzw. von ihr umhüllt, so daß die Peroxycarbonsäure den Kern der Kapseln bildet; es liegt somit ein Kapselsystem mit einer Kern/Hülle-Struktur vor. Die Kapseln können auch jeweils mehrere Kapselkerne aufweisen, insbesondere können sich im Rahmen der Herstellung Agglomerate aus Peroxycarbonsäureteilchen bilden. Eine Agglomeration mehrerer Kapseln ist ebenfalls möglich. Auf diese Weise kann sozusagen eine Matrix entstehen, in welche mehrere Kapselkerne eingebettet bzw. eingelagert sind.

Die Kapselhülle ist dabei auf Basis mindestens eines anorganischen Salzes ausgestaltet, wobei im Rahmen der vorliegenden Erfindung der Begriff "auf Basis mindestens eines anorganischen Salzes" so zu verstehen ist, daß neben dem anorganischen Salz auch noch weitere Materialien bzw. Substanzen, insbesondere wie nachfolgend angeführt, zur Ausbildung der Kapselhülle verwendet werden können. Entsprechendes gilt für den Kapselkern, der erfindungsgemäß auf Basis mindestens einer organischen Peroxycarbonsäure ausgestaltet ist und der ebenfalls weitere Substanzen - falls anwendungsbezogen erwünscht oder erforderlich - aufweisen kann.

Ein Verfahren zur Herstellung von mit mindestens einer organischen Peroxycarbonsäure, welche eine Imidoperoxycarbonsäure ist (z. B. PAP), beladenen Kapseln umfaßt insbesondere die folgenden Verfahrensschritte:
(a) Bereitstellung mindestens eines gegenüber der organischen Peroxycarbonsäure zumindest im wesentlichen inerten anorganischen Salzes, vorzugsweise in Form einer das anorganische Salz enthaltenden Lösung oder Dispersion; dann
(b) Aufbringen des in Verfahrensschritt (a) bereitgestellten anorganischen Salzes, insbesondere in Form der in Verfahrensschritt (a) bereitgestellten, das anorganische Salz enthaltenden Lösung oder Dispersion, auf die in Form fester Teilchen vorliegende organische Peroxycarbonsäure, insbesondere Imidoperoxycarbonsäure, so daß die Peroxycarbonsäure von einer Kapselhülle auf Basis des anorganischen Salzes zumindest im wesentlichen vollständig umhüllt bzw. beschichtet wird; anschließend
(c) gegebenenfalls Aufbereiten, insbesondere Trocknen und/oder Aufreinigen und/oder Formgebung und/oder Teilchengrößeneinstellung, der erhaltenen Kapseln.

Unter dem Begriff "Lösung" oder "Dispersion" kann ein flüssiges Milieu, insbesondere ein wäßriges Milieu, verstanden werden, welches das anorganische Salz vorzugsweise in dissoziierter Form enthält. Grundsätzlich kann aber in dem flüssigen Milieu das anorganische Salz aber auch in nichtgelöster oder nur teilweise gelöster, z. B. kristalliner Form, dispergiert vorliegen, beispielsweise wenn das Löslichkeitsprodukt des entsprechenden anorganischen Salzes in dem Medium überschritten ist.

Bei dem Verfahren werden als Substanzen, die in die Kapseln eingeschlossen werden, Imidoperoxycarbonsäuren, besonders bevorzugt 6-Phthalimidoperoxycapronsäure (6-Phthalimidoperoxyhexansäure, PAP) verwendet. Vorteilhafterweise sollte die Peroxycarbonsäure bei Atmosphärendruck, d. h. 101 325 Pa, einen Schmelzpunkt oberhalb von 25 °C, insbesondere oberhalb von 30 °C, vorzugsweise oberhalb von 45 °C, bevorzugt oberhalb von 50 °C, besonders bevorzugt oberhalb von 100 °C, aufweisen. Hierdurch ist gewährleistet, daß die verwendete Peroxycarbonsäure zumindest im wesentlichen in Form fester Teilchen vorliegt, so daß ein Abbau der Peroxycarbonsäuren während der Herstellung der Kapseln im Rahmen des Verfahrens zumindest weitgehend vermieden bzw. verringert wird und bei Anwendung des Kapselsystems eine kontrollierte Freisetzung erreicht wird.

Im Rahmen der vorliegenden Erfindung kann die Teilchengröße der eingesetzten organischen Peroxycarbonsäure 10 bis 3.000 µm, insbesondere 50 bis 2.500 µm, vorzugsweise 100 bis 1.500 µm betragen. In diesem Zusammenhang sollte die Teilchengröße der eingesetzten organischen Peroxycarbonsäure ≤ 3.000 µm, insbesondere ≤ 2.500 µm, vorzugsweise ≤ 2.250 µm, bevorzugt ≤ 2.000 µm, besonders bevorzugt ≤ 1.500 µm, sein. Dabei kann ein Einstellen der Teilchengröße der festen Peroxycarbonsäureteilchen - und der damit in Zusammenhang stehenden Größe der resultierenden Kapseln - vorzugsweise vor dem Auftragen der Kapselhülle in Verfahrensschritt (b) durch dem Fachmann als solche bekannte Verfahren durchgeführt werden, beispielsweise mittels Zerkleinern bzw. Vermahlen, Vibration und/oder Ultraschalleintrag, Absieben und dergleichen, so daß eine gezielte Anpassung der Teilchen- bzw. Kapselgröße entsprechend ihrer jeweiligen späteren Verwendung möglich ist.

Im allgemeinen sollten die weiteren Komponenten, welche in dem Verfahren zur Herstellung der Kapseln verwendet werden, derart ausgewählt sein, daß sie zumindest im wesentlichen kompatibel in bezug auf die zu verkapselnde bzw. zu beschichtende Peroxycarbonsäuren sind, d. h. es sollten keine unerwünschten chemischen Reaktionen, wie insbesondere Abbau-, Oxidations- bzw. Reduktionsreaktionen und/oder Hydrolysereaktionen, zwischen diesen Komponenten und der Peroxycarbonsäure und keine durch die weiteren Komponenten induzierten Reaktionen der Peroxycarbonsäure auftreten, welche zu ihrem Abbau, insbesondere Aktivitätsverlust, führen.

Das zur Herstellung der Kapselhülle eingesetzte anorganische Salz sollte ein nichtbasisches, vorzugsweise ein neutrales oder ein insbesondere schwach saures Salz sein. Das anorganische Salz kann dabei ausgewählt sein aus der Gruppe von anorganischen Sulfat-, Nitrat- und Phosphatsalzen, vorzugsweise anorganischen Sulfatsalzen. Beispielsweise kann das anorganische Salz ausgewählt sein aus Alkali- oder Erdalkalimetallsalzen, vorzugsweise Alkalimetallsalzen. Das anorganische Salz sollte dabei ein halogenidfreies Salz, insbesondere ein nichtbasisches, halogenidfreies anorganisches Salz, sein. Im Rahmen der vorliegenden Erfindung ist das anorganische Salz besonders bevorzugt Natriumsulfat. Das anorganische Salz ist insbesondere derart ausgewählt, daß es den pH-Wert der Lösung bzw. Dispersion, in welche die Kapseln überführt werden können (beispielsweise eine Wasch- oder Reinigungsmittelformulierung), zumindest im wesentlichen nicht erhöht bzw. nicht in Richtung des alkalischen Bereichs verschiebt, da dies die Stabilität der Peroxycarbonsäure herabsetzen kann. In diesem Zusammenhang sollten durch das anorganische Salz der Kapselhülle aber auch keine Chloridionen in die Lösung bzw. Dispersion der Kapseln eingebracht werden, da dies ebenfalls zu einer Verringerung der Stabilität der Peroxycarbonsäure führt. Vor diesem Hintergrund hat die Anmelderin herausgefunden, daß sich insbesondere Sulfatsalze, besonders bevorzugt Natriumsulfat, als Substanz für die Kapselhülle der erfindungsgemäßen Kapseln eignen und sich positiv auf die Stabilität der Peroxycarbonsäure auswirken. Für weitere Erläuterungen kann diesbezüglich auf die nachfolgenden Ausführungen verwiesen werden.

Der Gehalt an anorganischem Salz in der in Verfahrensschritt (a) bereitgestellten Lösung oder Dispersion zur Herstellung der Kapselhülle kann 0,1 bis 40 Gew.-%, insbesondere 1 bis 30 Gew.-%, vorzugsweise 5 bis 25 Gew.-%, bezogen auf die Lösung oder Dispersion, betragen. Der Gehalt an anorganischem Salz in der in Verfahrensschritt (a) bereitgestellten Lösung oder Dispersion sollte vorzugsweise mehr als 1 Gew.-%, insbesondere mehr als 5 Gew.-%, vorzugsweise mehr als 10 Gew.-%, bezogen auf die Lösung oder Dispersion, betragen. Dabei können der Lösung oder Dispersion auch weitere Substanzen zugegeben werden, welche in die Kapselhülle inkorporiert werden können. Dabei sollte auch hier gewährleistet sein, daß diese Substanzen weder den pH-Wert beeinflussen noch Halogenidionen, insbesondere Chloridionen, enthalten und weiterhin auch inert gegenüber der zu verkapselnden Peroxycarbonsäure sind. Beispielsweise können der Lösung oder Dispersion des anorganischen Salzes Komplex- bzw. Chelatbildner - insbesondere wie nachfolgend beschrieben - zugegeben werden.

Das Aufbringen des die Kapselhülle bildenden anorganischen Salzes kann durch Aufsprühen der Lösung bzw. Dispersion des anorganischen Salzes erfolgen. In diesem Zusammenhang kann in Verfahrensschritt (b) das Aufbringen der in Verfahrensschritt (a) bereitgestellten Lösung bzw. Dispersion durch Aufsprühen auf die in Form fester Teilchen vorliegende Peroxycarbonsäure, vorzugsweise in einer Wirbelschichtanlage oder mittels Sprühtrocknung (z. B. aus einer Aufschlämmung bzw. "Slurry" der Peroxycarbonsäure, insbesondere PAP, und Natriumsulfat), erfolgen. Alternativ kann das Aufbringen der Kapselhülle auch in einem Dragierkessel, Trommelcoater, Mischer bzw. Wurstercoater erfolgen.

Dabei sollte vorzugsweise unter Atmosphärendruck und/oder bei einer Temperatur von 0 bis 100 °C, insbesondere 10 bis 50 °C, vorzugsweise 20 bis 50 °C, verfahren werden. Es kann grundsätzlich auch bei erhöhten Drücken - sofern dies verfahrenstechnisch vorteilhaft oder erforderlich ist - gearbeitet werden. Im allgemeinen sollte die Temperatur derart ausgewählt sein, daß eine temperaturabhängige Zersetzung bzw. Auflösung der zu verkapselnden bzw. zu stabilisierenden organischen Peroxycarbonsäure zumindest im wesentlichen vermieden wird.

Bei dem Verfahren kann die Ausbildung der Kapselhülle aufgrund von physikalischen bzw. chemischen Wechselwirkungen oder Reaktionen, beispielsweise Ausfällungs-, Kristallisations- bzw. Kristallbildungsvorgängen, erfolgen. Dabei kann zur Ausbildung der Kapselhülle beispielsweise eine Kristallisation des gelösten bzw. dissoziierten anorganischen Salzes aus der Lösung oder Dispersion, vorzugsweise durch Ein- bzw. Verdampfen des auf die Peroxycarbonsäure aufgebrachten Löse- bzw. Dispersionsmittels, vorzugsweise Wasser, aber auch durch Abkühlen bzw. Ausfrieren erfolgen. Ohne sich auf eine Theorie festlegen zu wollen, kann durch diese Verfahrensschritte unter definierten Bedingungen die Sättigungskonzentration des anorganischen Salzes in der Lösung bzw. Dispersion überschritten werden, so daß eine Kristallbildung bzw. Kristallisation des anorganischen Salzes auf der Peroxycarbonsäure resultiert. Weiterhin kann eine Kristallbildung auch durch Ausfällungs- bzw. durch Aussalzungsvorgänge unterstützt bzw. hervorgerufen werden, wobei der Lösung oder Dispersion beispielsweise eine in bezug auf das anorganische Salz leichter lösliche Substanz oder ein weiteres Lösemittel für das anorganische Salz mit geringerer Löslichkeit, beispielsweise Ethanol, hinzugegeben werden kann.

Dabei kann im Rahmen der vorliegenden Erfindung die Kristallgröße des auf die Peroxycarbonsäure aufgebrachten anorganischen Salzes bzw. der resultierenden Kapselhülle in einem weiten Bereich eingestellt werden. So erhält man größere und regelmäßigere Kristalle, je langsamer ihr Wachstumsprozeß erfolgt (beispielsweise bei langsamen Abkühlen oder Ein- bzw. Verdampfen der Lösung bzw. Dispersion). Eine Kapselhülle mit feinkristalliner Struktur kann beispielsweise erhalten werden, indem man die Lösung oder Dispersion schnell abkühlt bzw. schnell verdampft.

Bei dem Verfahren kann auf die organische Peroxycarbonsäure, vorzugsweise vor Aufbringen der Kapselhülle, eine Substanz aufgebracht werden, welche bei einer Temperatur unterhalb von 80 °C, insbesondere unterhalb von 70 °C, endotherme Reaktionen mit sich selbst, beispielsweise Kristallwasserabspaltungsreaktionen oder Zersetzungsreaktionen, eingehen kann. Diese Substanz kann auch mit der Peroxycarbonsäure vermengt, insbesondere vermischt sein. Eine derartige Substanz ist beispielsweise Borsäure. Im Rahmen der vorliegenden Erfindung kann diese Substanz, vorzugsweise vor Aufbringen der Kapselhülle direkt auf die Peroxycarbonsäure aufgebracht werden, wobei beispielsweise dieselben Verfahrensschritte wie zur Bildung der Kapselhülle eingesetzt werden können. Die zugegebene Substanz führt zu einer Erhöhung der Handhabungssicherheit der Kapseln, da sie eine gegebenenfalls auftretende Wärmetönung abfangen bzw. kompensieren kann. Unter einer Wärmetönung kann eine in den Kapseln lokal auftretende Temperaturerhöhung, die durch eine lokal stattfindende bzw. beginnende exotherme Zersetzung der Peroxycarbonsäure hervorgerufen werden kann, aber auch eine in einem Gebinde bzw. in der Dispersion selbst - beispielsweise bei Lagerung - auftretende Temperaturerhöhung verstanden werden. Die zugegebene Substanz, beispielsweise Borsäure, kann auch in die das anorganische Salz enthaltende Kapselhülle eingebracht werden. Bevorzugt ist jedoch ein Aufbringen auf die Peroxycarbonsäure bzw. ein Vermengen bzw. Vermischen mit der Peroxycarbonsäure, da dies zu einer höheren Effektivität hinsichtlich der Handhabungssicherheit führt.

Weiterhin kann bei dem Verfahren der Kapselhülle mindestens ein Komplexbildner zugegeben werden. Dieser Komplexbildner kann beispielsweise ausgewählt sein aus der Gruppe von Chinolin und/oder seinen Salzen, Phosphaten, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP) und/oder Nitrilotriessigsäure (NTA). Diese Komplexbildner werden im Rahmen des Verfahrens insbesondere zur Komplexierung von Schwermetallionen eingesetzt, die andernfalls zu einem beschleunigten Abbau von Peroxycarbonsäuren führen.

Das in Verfahrensschritt (c) gegebenenfalls durchgeführte Trocknen kann durch übliche, dem Fachmann als solche bekannte Methoden erfolgen, beispielsweise durch Gefriertrocknung (Lyophilisierung), Verdampfen des Lösemittels, vorzugsweise bei einer Temperatur von 40 °C bis 60 °C, Ultrafiltration, Dialyse oder Sprühtrocknung unter schonenden Bedingungen. Eine in Verfahrensschritt (c) gegebenenfalls durchgeführte Formgebung kann beispielsweise durch Verrunden oder dergleichen erfolgen.

Die nach dem Verfahren erhaltenen, mit mindestens einer organischen Peroxycarbonsäure, nämlich Imidoperoxycarbonsäure, beladenen Kapseln können eine mittlere Größe (Kugeldurchmesser) von 10 bis 4.000 µm, vorzugsweise 50 bis 2.000 µm, bevorzugt 100 bis 1.000 µm, aufweisen. Gegebenenfalls kann, falls dies anwendungsbezogen erforderlich oder gewünscht sein sollte, eine Auftrennung der Kapseln entsprechend ihrer Größe (z. B. durch Klassierung) erfolgen, beispielsweise mittels Sieben.

Bei dem Verfahren beträgt der Anteil der Kapselhülle im allgemeinen ≥ 5 Gew.-%, insbesondere ≥ 10 Gew.-%, bevorzugt ≥ 20 Gew.-%, bezogen auf die Kapseln. Der Gehalt an anorganischer Peroxycarbonsäure, nämlich Imidoperoxycarbonsäure, vorzugsweise PAP, beträgt im allgemeinen 25 bis 95 Gew.-%, insbesondere 30 bis 90 Gew.-%, vorzugsweise 40 bis 85 Gew.-%, besonders bevorzugt 50 bis 80 Gew.-%, bezogen auf die Kapseln. Je nach Anwendung sollte dabei eine Anpassung des Gehalts an Peroxycarbonsäuren beispielsweise vor dem Hintergrund der Erhöhung der Handhabungssicherheit der erfindungsgemäßen Kapseln vorgenommen werden. So kann gegebenenfalls aus Gründen der Produktsicherheit ein allzu hoher Gehalt an Peroxycarbonsäure nicht wünschenswert sein. Für diese Fälle sollte der Gehalt an Peroxycarbonsäure beispielsweise bei etwa 50 Gew.-%, bezogen auf die Kapseln, liegen.

Die nach dem Verfahren hergestellten Kapseln weisen einen sogenannten "Controlled-released-Effekt" ("Effekt der kontrollierten Freisetzung") auf. Unter einem "Controlled-released-Effekt" ist insbesondere eine geringfügige, vorzugsweise zwischen 1 und 15 Minuten betragende, Verzögerung der Auflösung der Kapseln bei ihrer Anwendung, zum Beispiel in einer Waschflotte, bzw. eine Verzögerung der Freisetzung der Peroxycarbonsäure aus den Kapseln zu verstehen.

Im Rahmen des Verfahrens kann abschließend zusätzlich auf die Kapseln, welche eine Kapselhülle auf Basis des mindestens einen anorganischen Salzes aufweisen, mindestens eine zusätzliche Kapselhülle bzw. Hüllschicht aufgebracht werden. Dabei kann die zusätzliche Kapselhülle mindestens eine Substanz aufweisen, die ausgewählt sein kann aus anorganischen Salzen, insbesondere Sulfaten bzw. Phosphaten, anorganischen Oxiden, organischen Polymeren, beispielsweise Celluloseethern, Polyvinylalkoholen (PVAL) bzw. Polyvinylpyrrolidonen (PVP). Die zusätzliche Kapselhülle kann aber auch ein Gel auf Basis einer durch Zugabe mindestens eines Stabilisators, insbesondere Gelbildners, verfestigten bzw. gelierten Ölphase umfassen. Bevorzugt kann die zusätzliche Kapselhülle auch eine insbesondere mehrschichtige Polyelektrolytkapselhülle sein; beispielsweise kann die mehrschichtige Polyelektrolytkapselhülle mindestens zwei Schichten umfassen, die jeweils einen entgegengesetzt geladenen Polyelektrolyten bzw. jeweils entgegengesetzt geladene Polyionen aufweisen.

Durch das Aufbringen einer zusätzlichen Kapselhülle bzw. Hüllschicht kann ein zusätzlicher stabilisierender Effekt in bezug auf die zu stabilisierende organische Peroxycarbonsäure bewirkt werden. Beispielsweise kann hierdurch auch ein zusätzlicher "Controlled-release-Effekt" bezüglich der in den Kapseln enthaltenen Peroxycarbonsäuren erzielt werden. Die zusätzliche Hülle kann zu einer Unterdrückung bzw. Verminderung der Ab- bzw. Auflösung von Sulfat aus der Kapselhülle, beispielsweise in einer mit dem entsprechenden Salz nicht vollständig gesättigten Umgebung, führen und das Kapselsystem somit weiter stabilisieren. Das Aufbringen der zusätzlichen Hülle ("Coaten") kann in einer dem Fachmann an sich bekannten Weise, zum Beispiel mittels Wirbelschichtverfahren oder durch Adsorption des zusätzlichen Hüllmaterials ("Coatingmaterials") auf die Partikel aus einer Lösung, Aufsprühen einer Lösung oder Schmelze des Hüllmaterials auf die Partikel und anschließende Verdampfung des Lösemittels, vorzugsweise Wasser, oder mittels Umhüllung ("Coating") im Mischer, Kessel etc. durchgeführt werden. Auch in bezug auf die gegebenenfalls weiteren Hüllschichten ist die Zugabe weiterer Substanzen, beispielsweise Komplexbildnern und dergleichen, möglich, sofern es gewünscht bzw. erforderlich ist.

Die nach dem Verfahren in Verfahrensschritt (b) und/oder (c) erhaltenen Kapseln können in eine Dispersion überführt werden. Dabei sollte die Dispersion ein Dispersionsmittel, vorzugsweise Wasser, und ein anorganisches Salz umfassen, welches die Löslichkeit des die Kapselhülle bildenden anorganischen Salzes in der Dispersion herabsetzt. Das in der Dispersion gelöste bzw. dispergierte Salz kann dabei vorteilhafterweise dem die Kapselhülle bildenden Salz entsprechen. Der Gehalt an anorganischem Salz, besonders bevorzugt Natriumsulfat, sollte in der Dispersion derart gewählt werden, daß ein Abbau, insbesondere ein Ab- bzw. Auflösen, der die organische Peroxycarbonsäure umhüllenden bzw. beschichtenden Kapselhülle im Zustand der (konzentrierten) Dispersion zumindest im wesentlichen verhindert oder zumindest verringert wird. Unter der Formulierung, daß "das anorganische Salz der Dispersion dem anorganischen Salz der Kapselhülle entspricht", wird verstanden, daß vorzugsweise identische Salze, d. h. mit jeweils gleichem Kation bzw. Anion, für die Kapselhülle und die Dispersion der Kapseln verwendet werden. Darüber hinaus ist aber der Einsatz von solchen Salzen möglich, die eine Übereinstimmung in bezug auf mindestens einen ionischen Bestandteil aufweisen, d. h. identisch in bezug auf das jeweilige Kation oder identisch in bezug auf das jeweilige Anion sind. Weiterhin ist im allgemeinen das anorganische Salz der Dispersion so zu wählen, daß die Konzentrationen der entsprechenden Anionen bzw. Kationen derart in das Löslichkeitsprodukt des anorganischen Salzes der Kapselhülle einfließen, daß ein Ab- und/oder Auflösen der Kapselhülle in der Dispersion zumindest im wesentlichen verhindert oder zumindest verringert wird. Somit sollte die Art und Konzentration des anorganischen Salzes in der Dispersion derart gewählt werden, daß in der Dispersion die Sättigungskonzentration des anorganischen Salzes der Kapselhülle im wesentlichen erreicht bzw. vorzugsweise geringfügig unterschritten wird.

Der Gehalt an anorganischem Salz, besonders bevorzugt Natriumsulfat, in der Dispersion, insbesondere in dem Dispersionsmittel, kann beispielsweise derart gewählt werden, daß bei einer Temperatur, welche um 5 °C, insbesondere um 10 °C, vorzugsweise um 15 °C, geringer ist als die Lager- oder Aufbewahrungstemperatur der Dispersion, eine mit dem anorganischen Salz im wesentlichen gesättigte Dispersion erhalten wird. Der Gehalt an anorganischem Salz, besonders bevorzugt Natriumsulfat, sollte in diesem Zusammenhang derart gewählt werden, daß bei etwa 0 °C bis 15 °C, insbesondere 0 °C bis 10 °C, bevorzugt 0 °C bis 5 °C, im wesentlichen eine mit dem anorganischen Salz gesättigte Dispersion bzw. Kapseldispersion erhalten wird. Dabei kann der Gehalt an anorganischem Salz, besonders bevorzugt Natriumsulfat, in der Dispersion, insbesondere in dem Dispersionsmittel, 5 bis 30 Gew.-%, insbesondere 10 bis 30 Gew.-%, vorzugsweise 15 bis 30 Gew.-%, bezogen auf das Dispersionsmittel, betragen. Hierdurch soll im wesentlichen erreicht werden, daß ein Abbau, insbesondere ein Ab- und/oder Auflösen, der die organische Peroxycarbonsäure umhüllenden und/oder beschichtenden Kapselhülle auf Basis des mindestens einen anorganischen Salzes im Zustand der (konzentrierten) Dispersion zumindest im wesentlichen verhindert oder zumindest verringert wird.

Die zuvor definierte Dispersion kann erfindungsgemäß ein Wasch- und Reinigungsmittel, insbesondere ein flüssiges Wasch- und Reinigungsmittel, sein. Die in Verfahrensschritt (b) und/oder (c) erhaltenen Kapseln können erfindungsgemäß zusammen mit weiteren Inhaltsstoffen zu dem Wasch- oder Reinigungsmittel formuliert werden. Dabei sollte das Wasch- oder Reinigungsmittel zumindest im wesentlichen keine Halogenidionen, insbesondere Chloridionen aufweisen bzw. die Menge an Halogenidionen, insbesondere Chloridionen höchstens 500 ppm, vorzugsweise höchstens 100 ppm, besonders bevorzugt höchstens 30 ppm, betragen. Der pH-Wert sollte höchstens 7 betragen und insbesondere im Bereich von 3,5 bis 7, vorzugsweise von 4,0 bis 6, besonders bevorzugt von 4,5 bis 6, liegen. Ganz besonders bevorzugt sollte der pH-Wert etwa 5 betragen. Weiterhin kann das Wasch- oder Reinigungsmittel mindestens einen Komplexbildner enthalten; dieser kann beispielsweise ausgewählt sein aus der Gruppe von Chinolin und/oder seinen Salzen, Alkalimetallpolyphosphonaten, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren. Diese Komplexbildner werden im Rahmen des Verfahren insbesondere zur Komplexierung von Schwermetallionen eingesetzt. Weiterhin kann das Wasch- oder Reinigungsmittel gegebenenfalls mindestens ein wassermischbares Lösemittel mit einem für die organische Peroxycarbonsäure, insbesondere Imidoperoxycarbonsäure, geringen Lösungsvermögen aufweisen (z. B. in Mengen von vorzugsweise mehr als 20 Gew.-%, besonders bevorzugt mehr als 30 Gew.-%, bezogen auf das Wasch- oder Reinigungsmittel) oder aber das wassermischbare Lösemittel ist das Dispersionsmittel der Dispersion. Beispielsweise kann das Lösemittel Glycerin sein. Zudem kann dem Wasch- oder Reinigungsmittel mindestens ein Enzym, wie mindestens eine Katalase und/oder mindestens eine Peroxidase, vorzugsweise mindestens eine Katalase, und/oder mindestens ein Antioxidans zugesetzt werden. Zu weiteren Einzelheiten in bezug auf das Wasch- oder Reinigungsmittel kann auf nachfolgende Ausführungen verwiesen werden.

Das Verfahren dient gleichermaßen als ein Verfahren zur Stabilisierung von Peroxycarbonsäuren, nämlich Imidoperoxycarbonsäuren, vorzugsweise PAP, bzw. als ein Verfahren zur Erhöhung der Lagerfähigkeit von Peroxycarbonsäuren, nämlich Imidoperoxycarbonsäuren, vorzugsweise PAP.

Gemäß einer typischen Ausführungsform kann das Verfahren wie folgt durchgeführt werden: Zur Verkapselung bzw. zum Schutz der Peroxycarbonsäure, insbesondere Imidoperoxycarbonsäure (z. B. PAP), die in Form fester Teilchen, insbesondere in kristalliner Form, vorliegt, wird eine Kapselhülle aus einem nichtalkalischen, anorganischen Salz aufgebracht, so daß die Peroxycarbonsäure in einer tensid- und wasserhaltigen Flüssigmatrix sehr gut stabilisiert werden kann. Hierzu wird bevorzugt Natriumsulfat eingesetzt, der Anteil der Kapselhülle beträgt dabei mehr als 5 Gew.-%, bevorzugt mehr als 10 Gew.-%, besonders bevorzugt mehr als 20 Gew.-%, bezogen auf die Kapseln. Da ein lösliches Salz in einer herkömmlichen wasserbasierten Matrix spontan abgelöst wird, ist es vorteilhaft, der Flüssigmatrix ebenfalls eine große Menge vorzugsweise desselben Salzes, hier eine hohe Konzentration an Sulfat, zuzusetzen. Die Konzentration in der Flüssigmatrix sollte mehr als 5 Gew.-%, bevorzugt mehr als 10 Gew.-%, bezogen auf das gesamte System, betragen. In diesem Zusammenhang konnte die Anmelderin zeigen, daß eine vollständige Sättigung mit Sulfat bei Raumtemperatur nicht unbedingt erforderlich ist, um eine ausreichende Stabilität bzw. Lagerfähigkeit der Kapselhülle bzw. der Kapseln zu erhalten.

Weiterhin kann auf die Peroxycarbonsäure eine weitere Substanz aufgebracht werden. Auch kann vor dem Aufbringen der Kapselhülle die Peroxycarbonsäure mit einer weiteren Komponente vermischt werden, die durch eine bei erhöhter Temperatur auftretende endotherme Reaktion die Handhabungssicherheit der Kapseln erhöhen kann. Als solche Substanz eignet sich zum Beispiel Borsäure. Weiterhin kann der Kapselhülle ein Komplexbildner beigemischt sein. Als Komplexbildner eignen sich insbesondere solche, die bei einem leicht sauren pH-Wert (3 bis 6, bevorzugt 3,5 bis 5,5) wirksam sind. Beispielsweise eignen sich hierfür Phosphonate, zum Beispiel HDEP und DTPMP. Weiterhin kann - um die Sulfatkapselhülle in der Flüssigmatrix noch beständiger zu machen - eine weitere Kapselhülle (Umhüllung) aufgebracht werden. Als weitere Kapselhüllen eignen sich beispielsweise solche, die Polyelektrolytschichten mit alternierender Ladung bzw. Paraffingel umfassen. Diese führen dazu, daß die Ablösung des Sulfats in einer noch nicht ganz gesättigten Umgebung weiter unterdrückt bzw. zumindest verzögert wird.

Die nach dem Verfahren herstellbaren mit mindestens einer organischen Peroxycarbonsäure, nämlich imidoperoxycarbonsäure (z. B. PAP) beladenen Kapseln enthalten mindestens eine organische Peroxycarbonsäure, nämlich Imidoperoxycarbonsäure, vorzugsweise PAP, welche zumindest im wesentlichen mit einer Kapselhülle auf Basis eines anorganischen Salzes umhüllt bzw. beschichtet sind, wobei die Kapseln eine Kapselhülle auf Basis mindestens eines anorganischen Salzes und einen Kapselkern auf Basis mindestens einer organischen Peroxycarbonsäure, nämlich Imidoperoxycarbonsäure, umfassen.

Falls erforderlich oder anwendungsbezogen erwünscht, können dem Kernmaterial (= Peroxycarbonsäure) und/oder der Kapselhülle noch weitere Zusatz- oder Hilfsstoffe zugegeben sein, zum Beispiel Stabilisatoren, Modifizierungsmittel, anorganische Salze, Farbstoffe, Komplexbildner etc. Diesbezüglich kann auf obige Ausführungen verwiesen werden.

Für weitere Einzelheiten zu den Kapseln kann auf obige Ausführungen zu dem Verfahren verwiesen werden, die diesbezüglich entsprechend gelten.

Bei den Kapseln erfolgt der Abbau, insbesondere das Ab- bzw. Auflösen, der Kapselhülle bei der Anwendung der Kapseln (d. h. bei der Verdünnung der Dispersion) aufgrund von physikalischen bzw. chemischen Wechselwirkungen oder Reaktionen, beispielsweise Solubilisierungs- bzw. Dissoziationsvorgängen. Im Rahmen der vorliegenden Erfindung kann die Freisetzung der verkapselten Peroxycarbonsäuren, nämlich Imidoperoxycarbonsäure, beispielsweise PAP, aus den Kapseln, bei Verdünnung (z. B. in einer Waschflotte) erfolgen. Dabei kann die Freisetzung insbesondere durch einen Abbau, beispielsweise durch Ab- bzw. Auflösen bzw. Solubilisieren der Kapselhülle unter Verdünnung (z. B. in der Waschflotte) erfolgen. Diese Prozesse können insbesondere durch eine Verdünnung des in der Dispersion bzw. in dem flüssigen Wasch- oder Reinigungsmittel gelösten anorganischen Salzes hervorgerufen werden. Vorzugsweise ist die Verdünnung des anorganischen Salzes in der Dispersion dabei derart, daß das Löslichkeitsprodukt des die Kapselhülle bildenden anorganischen Salzes in der Dispersion bzw. in der Waschflotte derart unterschritten wird, daß ein Abbau der Kapselhülle, insbesondere durch Ab- bzw. Auflösen, erfolgen kann. Die Freisetzung der Peroxycarbonsäure kann dabei auch durch weitere Vorgänge, wie beispielsweise osmotische Prozesse und/oder Diffusionsvorgänge, hervorgerufen bzw. unterstützt werden. So können beispielsweise Wassermoleküle entlang des Konzentrationsgradienten durch die Kapselhülle in den Kernbereich des Kapselsystems diffundieren und dort bei einem entsprechenden pH-Wert der Waschflotte, insbesondere bei einem pH-Wert ≥ 7, zu einem An- bzw. Auflösen der Peroxycarbonsäure führen. Hierdurch kann ein Aufplatzen der Kapselhülle mit einhergehender Freisetzung der Peroxycarbonsäure in der Waschflotte bewirkt werden. Auch mechanische Einflüsse können zu einer Zerstörung der Kapselhülle beitragen, wie nachfolgend noch erläutert. Insbesondere ist auch eine Kombination der einzelnen vorgenannten Prozesse in bezug auf die Freisetzung der Peroxycarbonsäure aus dem Kapselsystem möglich.

Das Kapselsystem weist zahlreiche Verwendungsmöglichkeiten auf.

In diesem Zusammenhang können die Kapseln auch als "Delivery-System" bzw. "Controlled-release-System" zur kontrollierten Freisetzung von Peroxycarbonsäuren eingesetzt werden, wobei die Freisetzung der Peroxycarbonsäure insbesondere durch die Zusammensetzung und die Dicke der Kapselhülle kontrolliert werden kann. Unter Zusammensetzung wird hierbei insbesondere die Art und/oder Menge des entsprechenden anorganischen Salzes in der Kapselhülle verstanden. Eine weitere Modifikationsmöglichkeit stellt die Aufbringung mindestens einer zusätzlichen Kapselhülle (Umhüllung, "Coating") auf die Kapseln dar; ein solches Kapselsystem kann insbesondere auch als "Delivery-System" verwendet werden, bei dem die Peroxycarbonsäuren über einen großen Zeitraum durch eine verlängerte bzw. verzögerte Freisetzung abgegeben werden ("Sustained-release-Effekt").

Die erfindungsgemäßen Wasch- und Reinigungsmittel, welche das erfindungsgemäße Kapselsystem enthalten, sind sowohl im Haushalts- als auch im industriebereich einsetzbar.

Die erfindungsgemäßen Wasch- und Reinigungsmittel können zur Reinigung harter Oberflächen und/oder weicher, insbesondere textiler Oberflächen verwendet werden. Die erfindungsgemäßen Wasch- und Reinigungsmittel können insbesondere als Geschirrspülmittel, Allzweckreiniger, Badreiniger, Fußbodenreiniger, Autoreiniger, Glasreiniger, Möbelpflegemittel bzw. -reiniger, Fassadenreiniger, Waschmittel oder dergleichen verwendet werden, besonders bevorzugt als Waschmittel. Weiterhin sind die erfindungsgemäßen Wasch- und Reinigungsmittel vorzugsweise zur Reinigung von Fasern, Textilien, Teppichen und dergleichen geeignet.

Die erfindungsgemäßen Wasch- und Reinigungsmittel enthalten neben dem erfindungsgemäßen Kapselsystem an sich übliche Inhaltsstoffe bzw. Bestandteile (Tenside, Duftstoffe, Farbstoffe, Enzyme, Enzymstabilisatoren, Geruchsstoffe bzw. Builder, pH-Wert-Einstellmittel, andere Bleichmittel, Bleichaktivatoren, Silberschutzmittel, schmutzabweisende Substanzen, optische Aufheller, Vergrauungsinhibitoren, Desintegrationshilfsmittel, Verdickungsmittel, Entschäumer bzw. Schauminhibitoren, Komplexbildner für Schwermetalle, schmutzabweisende Substanzen bzw. Soil-Repellents, Farbübertragungsinhibitoren, Lösungsmittel, optische Aufheller und/oder weitere übliche Inhaltsstoffe), wobei im Rahmen der vorliegenden Erfindung auf die Kompatibilität der einzelnen Inhaltsstoffe bzw. Bestandteile sowohl untereinander als auch im Hinblick auf die erfindungsgemäßen Kapseln bzw. auf die darin verkapselten Peroxycarbonsäuren geachtet werden sollte, was durch gezielte Auswahl der Inhaltsstoffe bzw. Bestandteile und/oder ihrer jeweiligen Mengenverhältnisse realisiert wird. Auf diese Weise kann eine unerwünschte Wechselwirkung dieser Inhaltsstoffe bzw. Bestandteile mit den erfindungsgemäßen Kapseln bzw. den darin eingelagerten Peroxycarbonsäuren vermieden werden. Wie im folgenden noch näher ausgeführt, kann durch die gezielte Auswahl bestimmter Inhaltsstoffe bzw. Bestandteile und/oder deren Mengenverhältnisse ein stabilisierender. Effekt in bezug auf das erfindungsgemäße Kapselsystem bzw. die darin verkapselten Peroxycarbonsäuren bewirkt werden.

Ein erfindungsgemäßes flüssiges Wasch- oder Reinigungsmittel, umfaßt beispielsweise die folgenden Inhaltsstoffe:
(i) mit mindestens einer organischen Peroxycarbonsäure, nämlich Imidoperoxycarbonsäure, beladene Kapseln gemäß der vorliegenden Erfindung, vorzugsweise in Mengen von 0,1 bis 30 Gew.-%; und/oder
(ii) Tenside, insbesondere kationische und/oder anionische Tenside, vorzugsweise in Mengen von 0 bis 30 Gew.-%, und/oder nichtionische Tenside, vorzugsweise in Mengen von 0 bis 30 Gew.-%; und/oder
(iii) gegebenenfalls Elektrolyte, insbesondere anorganische und/oder organische Salze, insbesondere Phosphat, Citrat und/oder Sulfat, besonders bevorzugt Natriumsulfat, vorzugsweise in Mengen von 5 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, vorzugsweise in Mengen von 0 bis 10 Gew.-%; und/oder
(v) gegebenenfalls Enzyme, wie Proteasen, Amylasen, Cellulasen und/oder Lipasen, und/oder Enzymstabilisatoren, vorzugsweise in Mengen von 0 bis 10 Gew.-%; und/oder
(vi) gegebenenfalls Builder, insbesondere Fettsäuren, vorzugsweise gesättigte und/oder verzweigte Fettsäuren, insbesondere mit einem Schmelzpunkt unterhalb von 30 °C, und/oder Citronensäure und/oder Citrat, vorzugsweise in Mengen von 0 bis 15 Gew.-%; und/oder
(vii) gegebenenfalls Duftstoffe, vorzugsweise in Mengen von 0 bis 5 Gew.-%; und/oder
(viii) gegebenenfalls Hilfsstoffe, wie Entschäumer, pH-Regulatoren, Rheologiemodifikatoren (Verdicker), Lösungsmittel, Farbstoffe; und/oder
(ix) gegebenenfalls weitere übliche Inhaltsstoffe, wie Aufheller etc.; und/ oder
(x) Wasser;
wobei sämtliche Gewichtsangaben auf das Wasch- bzw. Reinigungsmittel bezogen sind.

Bei den erfindungsgemäßen flüssigen Wasch- bzw. Reinigungsmitteln sollte der Gehalt an anorganischem Salz, besonders bevorzugt Natriumsulfat, in der Dispersion (des flüssigen Wasch- oder Reinigungsmittels) derart gewählt werden, daß bei einer Temperatur, welche um 5 °C, insbesondere um 10 °C, vorzugsweise um 15 °C, geringer ist als die Lager- oder Aufbewahrungstemperatur (etwa 20 °C) der Dispersion, im wesentlichen eine mit dem anorganischen Salz gesättigte Dispersion vorliegt. In diesem Zusammenhang sollte der Gehalt an organischem Salz, besonders bevorzugt Natriumsulfat, derart gewählt werden, daß bei etwa 0 °C bis 15 °C, insbesondere 0 °C bis 10 °C, bevorzugt 0 °C bis 5 °C, im wesentlichen eine mit dem anorganischen Salz gesättigte Dispersion erhalten wird. Dabei kann der Gehalt an anorganischem Salz, besonders bevorzugt Natriumsulfat, in der Dispersion, insbesondere in dem Dispersionsmittel, 5 bis 30 Gew.-%, insbesondere 10 bis 30 Gew.-%, vorzugsweise 15 bis 30 Gew.-%, bezogen auf das Dispersionsmittel betragen. Für weitere diesbezügliche Einzelheiten kann auf obige Ausführungen verwiesen werden.

Denn die Anmelderin konnte zeigen, daß die Kapseln in ein bei Lager- bzw. Raumtemperatur mit dem anorganischem Salz, besonders bevorzugt Natriumsulfat, nicht vollständig gesättigten Wasch- bzw. Reinigungsmittel stabil eingearbeitet werden können, ohne daß die Kapselhülle abgebaut bzw. ab- oder aufgelöst wird. Es ist somit erfindungsgemäß nicht erforderlich - insbesondere vor dem Hintergrund der Rohstoffeinsparung und Produktionskostenminimierung - die Kapseln in eine mit dem der Kapselhülle entsprechenden Salz gesättigten Wasch- bzw. Reinigungsmittelformulierung einzubringen. Ohne sich auf eine spezielle Theorie festlegen zu wollen, beruht die Stabilität der erfindungsgemäßen Kapseln in einer solchen Formulierung auf einer sehr langsamen Kinetik der Abbauprozesse, insbesondere Solubilisierung und/oder Dissoziationsvorgänge, des die Kapselhülle bildenden anorganischen Salzes im Zustand der konzentrierten Dispersion.

Weiterhin sollte der Gehalt an anorganischem Salz, besonders bevorzugt Natriumsulfat, in dem Wasch- oder Reinigungsmittel derart gewählt sein, daß die Tenside in dem Wasch- oder Reinigungsmittel zumindest im wesentlichen inaktiviert sind, insbesondere durch Aussalzen, d. h. Induzierung einer Phasentrennung in eine tensidarme, kontinuierliche Phase und eine vorzugsweise lamellare, im allgemeinen hochviskose, kristalline oder flüssigkristalline tensidreiche Phase, vorzugsweise durch Einbringen mindestens einer Sulfatverbindung, besonders bevorzugt Natriumsulfat. Dabei wird in der Wasch- oder Reinigungsmittelformulierung insbesondere ein Auflösen der organischen Peroxycarbonsäure zumindest im wesentlichen verhindert oder zumindest verringert. Erfindungsgemäß wird unter dem Begriff "kontinuierliche Phase" das Dispersionsmittel mit den darin gelösten Bestandteilen oder Inhaltsstoffe (z. B. Salze, Tenside etc.) verstanden. Erfindungsgemäß bevorzugt ist das Dispersionsmittel Wasser.

Die Anmelderin konnte in diesem Zusammenhang zeigen, daß organische Peroxycarbonsäuren, insbesondere PAP, in Gegenwart von aktiven Tensiden (d. h. sich in freier oder micellarer Form in der Wasch- oder Reinigungsmittelformulierung befindlichen Tensiden) schnell zersetzt werden, da die Peroxycarbonsäuren durch die Tenside verstärkt gelöst werden und in diesem gelösten Zustand äußerst instabil sind. In diesem Zusammenhang führen insbesondere nichtionische Tenside bzw. Niotenside, beispielsweise auf Basis von Alkylpolyglykolethern, zu einer beschleunigten Zersetzung der Peroxycarbonsäuren. Durch die Zugabe von Sulfat werden die Tenside zumindest teilweise inaktiviert, was insbesondere durch Aussalzen geschieht, wobei die Tenside aus der insbesondere micellaren, aktiven Form in eine vorzugsweise lamellare, kristalline oder flüssigkristalline Form (Kristall- oder Flüssigkristallbildung) überführt werden, die in einer nahezu tensidfreien kontinuierlichen Phase dispergiert ist. Der dispergierte Flüssigkristall selbst, der beispielsweise durch Zentrifugation abgetrennt werden kann, soll dabei möglichst hochviskos sein. Der Gehalt an freien bzw. aktiven Tensiden in den erfindungsgemäßen Wasch- und Reinigungsmittelformulierungen bzw. in der kontinuierlichen Phase der erfindungsgemäßen Wasch- und Reinigungsmittelformulierungen allenfalls 1 % betragen.

Die Konzentration des anorganischen Salzes, besonders bevorzugt Natriumsulfat, in dem erfindungsgemäßen Wasch- oder Reinigungsmittel, sollte derart gewählt sein, daß bei der Anwendung des Wasch- oder Reinigungsmittels in der Waschflotte die Kapselhülle der erfindungsgemäßen Kapseln abgebaut, beispielsweise ab- bzw. aufgelöst werden, was beispielsweise durch einen Verdünnungseffekt beim Eintragen des Wasch- oder Reinigungsmittels in die Waschflotte erreicht werden kann. Dieser Verdünnungseffekt sollte derart ausgeprägt sein, daß - insbesondere bei Verwendung von Natriumsulfat als anorganisches Salz - bei der Anwendung des Wasch- oder Reinigungsmittels in der Waschflotte die Tenside wieder in aktiver Form vorliegen. Insbesondere sollte die Konzentration so gewählt sein, daß - wie zuvor erwähnt - in der kontinuierlichen Phase des nichtverdünnten Wasch- oder Reinigungsmittels weniger als 1 % gelöstes Tensid vorliegt und bei Temperaturabsenkung, insbesondere bei Temperaturabsenkungen bis auf 0 °C, keine Auskristallisation des Sulfats stattfindet.

Da insbesondere nichtionische Tenside hinsichtlich der Stabilität von Peroxycarbonsäure problematisch sein können, weisen die erfindungsgemäßen Wasch- und Reinigungsmittel ein entsprechend angepaßtes bzw. optimiertes Tensidverhältnis auf. Dabei sollte der Gehalt an Alkylpolyglykolethern möglichst gering sein.

Im Rahmen der vorliegenden Erfindung sollten die erfindungsgemäßen flüssigen Wasch- und Reinigungsmittel, zumindest im wesentlichen keine Halogenidionen, insbesondere keine Chloridionen aufweisen. Vorzugsweise beträgt die Menge an Halogenidionen, insbesondere Chloridionen, höchstens 500 ppm, vorzugsweise höchstens 100 ppm, besonders bevorzugt höchstens 30 ppm. Denn die Anmelderin hat überraschenderweise herausgefunden, daß eine hohe Halogenid-, insbesondere Chloridionenkonzentration, wie sie beispielsweise in herkömmlichen Wasch- und Reinigungsmitteln infolge von Verunreinigungen mancher Roh- bzw. Inhaltsstoffen üblich ist, zu einem verstärktem Abbau von Peroxycarbonsäuren führt. Somit kann eine Verringerung der Halogenidkonzentration, insbesondere der Chloridionenkonzentration, zu einem verminderten Abbau der Peroxycarbonsäure führen. Eine geringe Chloridionenkonzentration kann erfindungsgemäß beispielsweise durch den Einsatz von Methylsulfat-, Phosphat-, Tosylat- oder Cumolsulfonatverbindungen bzw. durch die Verwendung chloridarmer Roh- bzw. Inhaltsstoffe erreicht werden (z. B. Verwendung von im wesentlichen halogenidfreien Komponenten, so z. B. halogenidfreien Tensiden, halogenidfreien Phosphonaten etc.).

Weiterhin sollten die erfindungsgemäßen Wasch- und Reinigungsmittel einen pH-Wert von höchstens 7, insbesondere einen pH-Wert von 3,5 bis 7, vorzugsweise von 4,0 bis 6,5, besonders bevorzugt von 4,5 bis 6, ganz besonders bevorzugt von etwa 5, aufweisen. Bleichmittel auf Basis von Peroxycarbonsäuren, wie PAP, können überraschenderweise in einer sauren Umgebung, insbesondere bei einem pH-Wert ≤ 3,5, relativ wirkungsvoll stabilisiert werden, wohingegen bei neutralen oder alkalischen pH-Werten eine relativ schnelle Zersetzung von Peroxycarbonsäuren, wie PAP, stattfindet. Die Herabsetzung des pH-Wertes in den erfindungsgemäßen Wasch- und Reinigungsmitteln kann beispielsweise durch Zugabe von Säuren oder sauren Salzen erfolgen. Erfindungsgemäß bevorzugt sind Bisulfate, Bicarbonate und organische Polycarbonsäuren, die zum Beispiel gleichzeitig auch als Buildersubstanzen eingesetzt werden können. Ferner können die als Komplexbildner eingesetzten Phosphonate als Phosphonsäuren eingearbeitet werden und anschließend der gewünschte pH-Wert durch Zugabe von Alkalien eingestellt werden.

Die erfindungsgemäßen Wasch- oder Reinigungsmittel können mindestens eine Fettsäure enthalten. Dabei sind gesättigte und/oder verzweigte Fettsäuren, insbesondere mit einem Schmelzpunkt unterhalb von 30 °C, erfindungsgemäß bevorzugt. Im Rahmen der vorliegende Erfindung kann beispielsweise Isocarb-16^{®} der Firma Sasol in den erfindungsgemäßen Wasch- oder Reinigungsmitteln eingesetzt werden.

Die erfindungsgemäßen Wasch- oder Reinigungsmittel weisen einen optimierten Citronensäure- bzw. Citratgehalt auf. Wie die Anmelderin herausgefunden hat, kann Citronensäure bzw. Citrat zu einem Abbau von Peroxycarbonsäuren, insbesondere PAP, führen. Dennoch kann es gegebenenfalls erforderlich sein, Citronensäure bzw. Citrate in dem Wasch- oder Reinigungsmittel bzw. in dem Dispersionsmittel für die erfindungsgemäßen Kapseln einzusetzen (zum Beispiel als Builder und/oder als Komplexbildner). Die verwendeten Mengen sollten dabei jedoch nicht zu hoch sein und in bezug auf die Peroxycarbonsäuren, insbesondere PAP, angepaßt sein.

Zudem kann das erfindungsgemäße Wasch- oder Reinigungsmittel mindestens einen Komplexbildner enthalten, der insbesondere ausgewählt sein kann aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettigen Dicarbonsäuren. Weitere Beispiele für erfindungsgemäß verwendbare Komplexbildner für Schwermetalle sind zum Beispiel Aminopolycarbonsäuren, Aminohydroxypolycarbonsäuren, Polyphosphonsäuren und Aminopolyphosphonsäuren. Diese Komplexbildner werden erfindungsgemäß eingesetzt, um Schwermetallionen zu inaktivieren bzw. zu binden, die insbesondere als Katalysatoren von Oxidationsprozessen fungieren und somit zu einem Abbau von Peroxycarbonsäuren, wie PAP, führen können und die beispielsweise über Wasserleitungen oder metallische Bauteile der Produktionsanlagen oder über Roh- bzw. Inhaltsstoffe in das erfindungsgemäße Wasch- oder Reinigungsmittel eingetragen werden können.

Darüber hinaus können die erfindungsgemäßen Wasch- und Reinigungsmittel mindestens ein wassermischbares Lösemittel mit einem für die organische Peroxycarbonsäuren geringen Lösungsvermögen enthalten, wie vorzugsweise Glycerin.

Weiterhin können die erfindungsgemäßen Wasch- und Reinigungsmittel mindestens eine Katalase enthalten, um gegebenenfalls durch Umsetzung der Peroxycarbonsäure mit Wasser entstehendes Wasserstoffperoxid wirkungsvoll aus der kontinuierlichen Phase des Produktes, insbesondere des Wasch- oder Reinigungsmittels, zu entfernen, so daß insbesondere die dort gegebenenfalls vorhandenen Enzyme wirkungsvoll vor Oxidationsprozessen, die gegebenenfalls zu einem Aktivitätsverlust der Enzyme führen können, geschützt werden. Zu diesem Zweck können den erfindungsgemäßen Wasch- oder Reinigungsmitteln gleichermaßen mindestens eine Peroxidase und/oder mindestens ein Antioxidans, gegebenenfalls zusätzlich zu der mindestens einen Katalase, zugegeben werden. Erfindungsgemäß bevorzugte Antioxidantien sind beispielsweise Ascorbinsäure, Tocopherol, Gallussäure oder deren Derivate.

Weiterhin sollte die erfindungsgemäße Wasch- oder Reinigungsmittelformulierung derart gestaltet sein, daß sie die erfindungsgemäßen Kapseln insbesondere im wesentlichen nicht an- bzw. auflösen. Im allgemeinen sollten die Komponenten, welche in dem erfindungsgemäßen Wasch- oder Reinigungsmittel eingesetzt werden, derart ausgewählt sein, daß sie zumindest im wesentlichen kompatibel in bezug auf die erfindungsgemäßen Kapseln sind, d. h. es sollten insbesondere in dem Wasch- oder Reinigungsmittel selbst, insbesondere in dem Zeitraum vor seiner Anwendung (Lagerzeit), keine unerwünschten chemischen Reaktionen, wie insbesondere Abbau-, Oxidations- bzw. Reduktionsreaktionen und/oder Hydrolysereaktionen, zwischen diesen Komponenten und den Kapseln auftreten, welche zu einem vorzeitigen Abbau und zu einem Aktivitätsverlust der Peroxycarbonsäuren führen.

Um in der Waschflotte eine ausreichende Bleichleistung zu erzielen, sollte die Peroxycarbonsäure aus den erfindungsgemäßen Kapseln ausreichend schnell freigesetzt werden. Die Freisetzung der Peroxycarbonsäure erfolgt bei der Anwendung des Wasch- oder Reinigungsmittels, insbesondere durch physikalische bzw. durch physiko-chemische oder chemische Prozesse, beispielsweise durch einen Abbau, insbesondere Ab- oder Auflösen, der Kapselhülle in der Waschflotte, und durch osmotische Prozesse bzw.

Diffusionsvorgänge. Die Freisetzung der verkapselten Peroxycarbonsäuren, nämlich Imidoperoxycarbonsäure, beispielsweise PAP, aus den Kapseln, insbesondere in einer Waschflotte, kann durch einen Abbau, beispielsweise durch An- bzw. Auflösen bzw. Solubilisieren, der Kapselhülle erfolgen. Diese Prozesse können insbesondere durch einen Verdünnungseffekt hervorgerufen werden. Vorzugsweise ist die Verdünnung des anorganischen Salzes in der Dispersion bzw. in der Waschflotte dabei derart, daß das Löslichkeitsprodukt des die Kapselhülle bildenden anorganischen Salzes in der Dispersion bzw. in der Waschflotte unterschritten wird, so daß ein Abbau der Kapselhülle, insbesondere ein Ab- bzw. Auflösen, erfolgen kann.

Die Freisetzung der Peroxycarbonsäure kann dabei auch durch weitere Prozesse hervorgerufen bzw. unterstützt werden. So führt eine Verdünnung des anorganischen Salzes, besonders bevorzugt Natriumsulfat, in der Dispersion bzw. Waschflotte gleichzeitig zu einer Überführung der Tenside aus ihrer inaktiven Form (wie sie z. B. durch Aussalzen beispielsweise in Form von Flüssigkristallen in der nichtverdünnten Wasch- oder Reinigungsmittelformulierung vorliegen) in die aktive, gelöste bzw. micellare Form, so daß die auf diese Weise aktivierten Tenside die Peroxycarbonsäure an- und/oder auflösen können bzw. die Solubilisierung der Kapselhülle unterstützen können. Bei dem Verdünnen in der Waschflotte tritt gleichzeitig ein deutlicher pH-Wert-Sprung des im allgemeinen sauer eingestellten Wasch- und Reinigungsmittels ein, so daß auch hierdurch die Löslichkeit der Peroxycarbonsäure deutlich zunimmt.

Darüber hinaus können Diffusionsprozesse auftreten (zum Beispiel Diffusion von Wassermolekülen entlang des Konzentrationsgradienten durch die Kapselhülle in den Kernbereich des Kapselsystems, so daß dort bei einem entsprechenden pH-Wert der Waschflotte, insbesondere bei einem pH-Wert ≥ 7, die Peroxycarbonsäure an- bzw. aufgelöst werden kann). Hierdurch kann auch - sofern die Kapselhülle nicht vollständig abgebaut ist - ein hoher osmotischer Druck in den Kapseln generiert werden, der sozusagen zu einem Aufplatzen der Kapselhülle mit einhergehender Freisetzung der Peroxycarbonsäure in der Waschflotte führen kann. Schließlich spielen auch mechanische Vorgänge eine Rolle, zum Beispiel eine mechanische Zerstörung der Kapseln durch die in der Waschflotte befindlichen Wäschestücke bzw. durch Kontakt mit der Waschtrommel. Wie zuvor erläutert, resultiert aus der besonderen Struktur bzw. dem Aufbau der erfindungsgemäßen Kapseln eine kontrollierte bzw. verzögerte Freisetzung der Peroxycarbonsäure. Insbesondere ist auch eine Kombination der einzelnen vorgenannten Prozesse in bezug auf die Freisetzung der Peroxycarbonsäure aus den erfindungsgemäßen Kapseln möglich.

Die vorliegende Erfindung zeigt gegenüber dem Stand der Technik eine Reihe von Vorteilen auf:

Die Ausbildung der Kapselhülle erfolgt aufgrund von physikalisch-chemischen bzw. physikalischen Wechselwirkungen, so daß für die Ausbildung der Kapselstruktur keine Polymerisationsschritte, insbesondere radikalische Polymerisationsschritte, notwendig sind, wie dies in einigen Verfahren des Standes der Technik der Fall ist. Derartige Polymerisationen führen häufig zur Zersetzung des Aktiv- und/oder Wirkstoffs, insbesondere der empfindlichen Peroxycarbonsäure.

In diesem Zusammenhang ist es insbesondere vorteilhaft, daß das Verhältnis der Kapselhülle auf Basis eines anorganischen Salzes in bezug auf den Gehalt an anorganischer Peroxycarbonsäure angepaßt werden kann, so daß ein Maßschneidern der Kapseln in bezug auf die Empfindlichkeit der zu verkapselnden Peroxycarbonsäuren erreicht werden kann. Durch die gezielte Einstellbarkeit der Kapselgröße kann eine effektive Dosierung des Wirkstoffs ermöglicht werden. Darüber hinaus können Kapseln mit einem hohen Wirkstoffgehalt realisiert werden, so daß für die Kapselhüllen nur wenig Ausgangssubstanz verwendet werden muß.

Im Gegensatz zu Verkapselungssystemen, die beispielsweise auf Wachsen basieren, enthalten die erfindungswesentlichen Kapseln keine störenden Kapselhüllen, die im Rahmen des Waschprozesses zu unerwünschten Rückständen auf der Wäsche führen. Die aus dem anorganischen Salz bestehende Kapselhülle wird in der Waschflotte einfach aufgelöst und durch Spülprozesse nahezu vollständig entfernt.

Durch den Einsatz des anorganischen Salzes, besonders bevorzugt Natriumsulfat, in der Wasch- oder Reinigungsformulierung wird die Stabilisierung der Peroxycarbonsäure einerseits durch eine Inaktivierung der Tenside und andererseits durch eine weitgehende Verhinderung des Abbaus der Kapselhülle ermöglicht, so daß das eingesetzte anorganische Salz diesbezüglich eine doppelte Funktion erfüllt. Der Einsatz weiterer Substanzen, beispielsweise zur Inaktivierung von Tensiden, kann daher entfallen.

Durch das Herstellungsverfahren und die auf diese Weise erhältlichen Kapseln ist gewährleistet, daß die Kapseln einerseits aus wäßrigen Lösungen bzw. Dispersionen heraus aufgebracht werden können und andererseits die Kapseln in einer wasserhaltigen flüssigen Formulierung, wie beispielsweise einem flüssigen Wasch- oder Reinigungsmittel, nicht an- bzw. aufgelöst werden.

Weiterhin kann durch die gezielte Modifizierung des Kapselkerns bzw. der Kapselhülle, beispielsweise durch Zugabe von Komplexbildnern für Schwermetallionen, eine weitere Verbesserung des Schutzes der Peroxycarbonsäure und damit einhergehend eine weitere Erhöhung ihrer Lagerstabilität erreicht werden. Durch das Aufbringen weiterer Kapselhüllen kann die Stabilität, insbesondere Lagerstabilität, der Peroxycarbonsäure weiter erhöht werden, so daß eine hervorragende Anpassungsfähigkeit des Kapselsystems im Hinblick auf die jeweilige Anwendung vorliegt.

Insbesondere eignen sich die Kapseln zur Einarbeitung bzw. Anwendung in Tenside enthaltenden Systemen, beispielsweise tensidischen (tensidhaltigen) Dispersionen für flüssige Wasch- und Reinigungsmittel. Dies ist ein besonderer Vorteil, da die unverkapselten bzw. ungeschützten Peroxycarbonsäuren, insbesondere PAP, in Gegenwart von Tensiden nicht stabil sind und rasch zersetzt werden, so daß ihr Einsatz in tensidhaltigen flüssigen, insbesondere wäßrigen Medien bislang nicht möglich bzw. allenfalls sehr beschränkt möglich war. Der stabilisierende Effekt der Kapseln, der zusätzlich mit einer gewünschten kontrollierten Freisetzung der verkapselten Peroxycarbonsäure verbunden ist, kann in synergistischer Weise dadurch gesteigert werden, daß das Medium, in dem sich die Kapseln befinden, derart eingestellt wird, daß es eine zusätzliche Stabilisierung in bezug auf die Peroxycarbonsäuren liefert, insbesondere durch Inaktivierung der Tenside, Optimierung bzw. Absenkung des pH-Wertes, Reduzierung des Halogenidgehaltes, Verwendung eines Lösemittels mit geringem Lösungsvermögen in bezug aus Peroxycarbonsäuren und dergleichen.

Die Kapseln lassen sich insbesondere in flüssige Wasch- und Reinigungsmittel stabil einarbeiten. Eine zusätzliche Verhinderung bzw. Verringerung der Kapselsedimentation kann zum Beispiel durch geeignete, dem Fachmann an sich bekannte Verdickersysteme erreicht werden. Die Kapseln verfügen dort über eine hohe Lagerstabilität und können auch nach größeren Zeiträumen die Peroxycarbonsäure wirkungsvoll freisetzen.

Die erfindungsgemäßen Wasch- und Reinigungsmittelformulierungen weisen aufgrund ihrer zuvor angeführten, aufeinander abgestimmten und synergistisch wirkenden Modifikationen, d. h. Anpassung der Formulierung, wie insbesondere geringe Halogenidionengehalt, Optimierung des pH-Wertes, Zugabe von Komplexbildnern, Inaktivierung der Tenside, speziellen Lösemitteln bzw. Enzymen, wie Katalasen oder Peroxidasen, Zugabe von Antioxidantien, gegenüber dem Stand der Technik erhebliche Vorteile auf, da in Verbindung mit den erfindungsgemäßen Kapseln der Abbau der empfindlichen Bleichmittel auf Peroxycarbonsäurebasis deutlich vermindert wird.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung, die in den Ansprüchen angegeben wird, verläßt.

Die vorliegende Erfindung wird anhand der folgenden Ausführungsbeispiele verdeutlicht, welche die Erfindung jedoch keinesfalls beschränken:

### Ausführungsbeispiele:

### Beispiel 1: Herstellung von verkapselten bzw. beschichteten Bleichmittelkapseln mit einer Sulfatkapselhülle

800 g Eureco^{®} W (Fa. Solvay) wurden in einer Labor-Wirbelschichtanlage (Aeromatic^{®}) mit 800 g einer 20%igen Natriumsulfatlösung besprüht, der 1 % Sequion^{®} 10H60 zugesetzt wurde. Das erhaltene Produkt wurde auf < 2,0 mm abgesiebt.

Mit Hilfe einer iodometrischen Titration wurde der Aktivstoffgehalt (reines PAP) bestimmt. Eswurde ein Wert von 70,5 % erhalten.

### Beispiel 2: Stabilitätstest

Das in Beispiel 1 hergestellte gecoateten Bleichmittelcompound wurde in folgende Flüssigrezeptur eingearbeitet (die Prozentwerte sind Aktivstoffangaben):

| | |
|---|---|
| Dehydol^{®} LT7 (Fa. Cognis) | 4,0 % |
| LAS (Maranil^{®} A 55) (Fa. Cognis) | 22,5 % |
| Na₂SO₄ | 12,5 % |
| Sequion^{®} 10H60 (Fa. Polygon Chemie AG) | 1,0 % |
| Xanthan Gum (Jungbunzlauer) | 0,4 % |
| Erfindungsgemäße Kapseln | 4,3 % |
| Wasser | ad 100,0 % |

Der pH-Wert wurde mit Natronlauge auf 5,0 eingestellt.

Vergleichsbeispiel: Es wurden 3,5 % Eureco^{®} W unbehandelt eingearbeitet. Der pH-Wert betrug ebenfalls 5,0.

Durch eine iodometische Titration wurden die Aktivsauerstofferhaltungsgrade (100 % bei Lagerbeginn) nach verschiedenen Lagerzeiten bei 40 °C bestimmt.

### Ergebnisse:

| | Erfindungsgemäß | Vergleichsbeispiel |
|---|---|---|
| 1 Woche | 99 % | 75% |
| 2 Wochen | 95 % | 65% |

Man erkennt, daß die Stabilität des PAP in den Kapseln deutlich erhöht ist.

### Beispiel 3:

Rezeptur für eine weitere Flüssigformulierung, in der die verkapselten bzw. beschichteten Bleichmittelkapseln mit einer Sulfatkapselhülle eingelagert werden können:

| | |
|---|---|
| LAS (Maranil^{®} A 55) | 18,5 % |
| Dehydol^{®} LT 7 (Fa. Cognis) | 8 % |
| Natriumsulfat | 11 % |
| Xanthan-Gummi | 0,4 % |
| Sequion^{®} 10 H 60 (Fa. Polygon Chemie AG) | 1 % |
| Silikon-Entschäumer | 0,2 % |
| Kapselsystem aus Beispiel 1 | 3 % |
| Wasser | ad 100 % |

## Patentansprüche

1. Flüssige Wasch- und Reinigungsmittelzusammensetzüngen, enthaltend Dis-persionen, insbesondere wäßrige Dispersionen, enthaltend mit mindestens einer organischen Peroxycarbonsäure, welche eine Imidoperoxycarbonsäure, vorzugsweise PAP, ist, beladene Kapseln, umfassend mindestens eine Imidoperoxycarbonsäure, vorzugsweise PAP, welche zumindest im wesentli-chen mit einer Kapselhülle auf Basis eines anorganischen Salzes umhüllt und/oder beschichtet sind, wobei die Kapseln eine Kapselhülle auf Basis mindestens eines anorganischen Salzes und einen Kapselkern auf Basis mindestens einer Imidoperoxycarbonsäure umfassen, und ein anorganisches Salz, welches die Löslichkeit des die Kapselhülle bildenden anorganischen Salzes in der Dispersion herabsetzt.

2. Mittel nach Anspruch 1, **dadurch gekennzeichnet, daß** die Imidoperoxycarbonsäure 6-Phthalimidoperoxycapronsäure (6-Phthalimido-peroxyhexansäure, PAP) ist.

3. Mittel nach Anspruch 1 oder 2, **dadurch gekennzeichnet, daß** das anorganische Salz ein nichtbasisches, vorzugsweise ein neutrales oder ein insbesondere schwach saures Salz ist und/oder daß das anorganische Salz ausgewählt ist aus der Gruppe von anorganischen Sulfat-, Nitrat- und Phosphatsalzen, vorzugsweise anorganischen Sulfatsalzen, insbesondere wobei das anorganische Salz ausgewählt ist aus Alkali- oder Erdalkalimetallsalzen, vorzugsweise Alkalimetallsalzen, und/oder daß das anorganische Salz ein halogenidfreies Salz, insbesondere ein nichtbasisches, halogenidfreies anorganisches Salz, ist, und/oder daß das anorganische Salz Natriumsulfat ist.

4. Mittel nach einem der Ansprüche 1 bis 3, **dadurch gekennzeichnet, daß** auf die organische Peroxycarbonsäure eine Substanz, welche bei einer Temperatur unterhalb von 80 °C, insbesondere unterhalb von 70 °C, vorzugsweise unterhalb von 60 °C, endotherme Reaktionen, insbesondere Kristallwasserabspaltungsreaktionen und/oder Zersetzungsreaktionen, eingehen kann, aufgebracht ist und/oder mit der Peroxycarbonsäure vermengt, insbesondere vermischt, ist, insbesondere wobei die Substanz vorzugsweise Borsäure ist, und/oder daß die Kapselhülle mindestens einen Komplexbildner umfaßt, insbesondere ausgewählt aus der Gruppe von Chinolin und/oder seinen Salzen, Phosphaten, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methyl-enphosphonsäure) (DTPMP), Azacycloheptandiphosphonat (AHP) und/oder Nitrilotriessigsäure (NTA).

5. Mittel nach einem der Ansprüche 1 bis 4, **dadurch gekennzeichnet, daß** die Teilchengröße der Imidoperoxycarbonsäure 10 bis 3.000 µm, insbesondere 50 bis 2.500 µm, vorzugsweise 100 bis 1.500 µm, beträgt und/oder daß die Teilchengröße der eingesetzten Imidoperoxycarbonsäure ≤ 3.000 µm, insbesondere ≤ 2.500 µm, vorzugsweise ≤ 2.250 µm, bevorzugt ≤ 2.000 µm, besonders bevorzugt ≤ 1.500 µm, ist und/oder daß die mit mindestens einer Imidoperoxycarbonsäure beladenen Kapseln eine mittlere Größe (Kugeldurchmesser) von 10 bis 4.000 µm, vorzugsweise 50 bis 2.000 µm, bevorzugt 100 bis 1.000 µm, aufweisen.

6. Mittel nach einem der Ansprüche 1 bis 5, **dadurch gekennzeichnet, daß** der Anteil der Kapselhülle ≥ 5 Gew.-%, insbesondere ≥ 10 Gew.-%, bevorzugt ≥ 20 Gew.-%, bezogen auf die Kapseln, ist und/oder daß der Gehalt an Imidoperoxycarbonsäure, vorzugsweise PAP, 25 bis 95 Gew.-%, insbesondere 30 bis 90 Gew.-%, vorzugsweise 40 bis 85 Gew.-%, besonders bevorzugt 50 bis 80 Gew.-%, bezogen auf die Kapseln, beträgt.

7. Mittel nach einem der Ansprüche 1 bis 6, **dadurch gekennzeichnet, daß** auf die Kapselhülle mindestens eine zusätzliche Kapselhalle aufgebracht ist.

8. Mittel nach einem der Ansprüche 1 bis 7, **dadurch gekennzeichnet, daß** es umfaßt:
(i) mit Imidoperoxycarbonsäure beladene Kapseln in Mengen von 0,1 bis 30 Gew.-%; und/oder
(ii) Tenside, insbesondere kationische und/oder anionische Tenside, vorzugsweise in Mengen von 0 bis 30 Gew.-%, und/oder nichtionische Tenside, vorzugsweise in Mengen von 0 bis 30 Gew.-%; und/oder
(iii) gegebenenfalls Elektrolyte, insbesondere anorganische und/oder organische Salze, insbesondere Phosphat, Citrat und/oder Sulfat, besonders bevorzugt Natriumsulfat, vorzugsweise in Mengen von 5 bis 30 Gew.-%; und/oder
(iv) gegebenenfalls Komplexbildner, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsaure) (DTPMP), Azacycloheptandiphosphonat (AHP), Nitrilotriessigsäure (NTA), Citrat und/oder kurzkettige Dicarbonsäure, vorzugsweise in Mengen von 0 bis 10 Gew.-%; und/oder
(v) gegebenenfalls Enzyme und/oder Enzymstabilisatoren, vorzugsweise in Mengen von 0 bis 10 Gew.-%; und/oder
(vi) gegebenenfalls Builder, insbesondere Fettsäuren und/oder Citronensäure und/oder Citrat, vorzugsweise in Mengen von 0 bis 15 Gew.-%; und/oder
(vii) gegebenenfalls Duftstoffe, vorzugsweise in Mengen von 0 bis 5 Gew.%; und/oder
(viii) gegebenenfalls Hilfsstoffe, wie Entschäumer, pH-Regulatoren, Rheologiemodifikatoren (Verdicker), Lösungsmittel, Farbstoffe; und/oder
(ix) gegebenenfalls weitere übliche inhaltsstoffe, wie Aufheller etc.; und/oder
(x) Wasser;
wobei sämtliche Gewichtsangaben auf das Wasch- bzw. Reinigungsmittel bezogen sind.

9. Mittel nach einem der Ansprüche 1 bis 8, **dadurch gekennzeichnet, daß** es
- zumindest im wesentlichen keine Halogenidionen, insbesondere Chloridionen, aufweist, insbesondere wobei die Menge an Halogenidionen, insbesondere Chloridionen, höchstens 500 ppm, vorzugsweise höchstens 100 ppm, besonders bevorzugt höchstens 30 ppm, beträgt; und/oder
- einen pH-Wert von höchstens 7, insbesondere einen pH-Wert von 3,5 bis 7, vorzugsweise von 4,0 bis 6,5, besonders bevorzugt von 4,5 bis 6, aufweist; und/oder
- mindestens einen Komplexbildner enthält, insbesondere ausgewählt aus der Gruppe von Chinolin und/oder seinen Salzen, Alkalimetallpolyphosphonaten, Picolinsäure und Dipicolinsäure, Mono- oder Polyphosphonsäuren, insbesondere 1-Hydroxyethyliden-1,1-diphosphonsäure (HEDP), Ethylendiamintetraessigsäure (EDTA), Diethylentriaminpenta(methylenphosphonsäure) (DTPMP), Azacycloheptandiphosphonat (AHP), Nitrilotriessigsäure (NTA), Citrat und/oder kurzkettige Dicarbonsäuren, insbesondere zur Komplexierung von Schwermetallionen; und/oder
- gegebenenfalls mindestens ein wassermischbares Lösemittel mit einem für die Imidoperoxycarbonsäure geringen Lösungsvermögen, vorzugsweise Glycerin, enthält; und/oder
- gegebenenfalls mindestens eine Katalase enthält;
so daß ein Abbau der Imidoperoxycarbonsäure, vorzugsweise PAP, in dem Wasch- oder Reinigungsmittel zumindest vermindert oder verhindert wird.

## Claims

1. Liquid washing and cleaning agent compositions, comprising dispersions, in particular aqueous dispersions, comprising capsules loaded with at least one organic peroxycarboxylic acid that is an Imidoperoxycarboxylic acid, preferably PAP, containing at least one Imidoperoxycarboxylic acid, preferably PAP, and which are at least substantially coated and/or encased with a capsule shell based on an inorganic salt, wherein the capsules contain a capsule shell based on at least one inorganic salt and a capsule core based on at least one Imidoperoxycarboxylic acid, and an inorganic salt that lowers the solubility of the inorganic salt that forms the capsule shell in the dispersion.

2. Agent according to claim 1, **characterised in that** the Imidoperoxycarboxylic acid is 6-phthalimidoperoxycaproic acid (6-phthalimidoperoxyhexanoic acid, PAP).

3. Agent according to claim 1 or 2, **characterised in that** the inorganic salt is a non-basic salt, preferably a neutral salt or a particularly weakly acidic salt, and/or **in that** the inorganic salt is selected from the group of inorganic sulfate, nitrate and phosphate salts, preferably inorganic sulfate salts, in particular wherein the inorganic salt is selected from alkali or alkaline earth metal salts, preferably alkali metal salts, and/or **in that** the inorganic salt is a halide-free salt, in particular a non-basic, halide-free, inorganic salt, and/or in that the inorganic salt is sodium sulfate.

4. Agent according to one of claims 1 to 3, **characterised in that** a substance that at a temperature below 80 °C, in particular below 70 °C, preferably below 60 °C, may undergo endothermic reactions, in particular water of crystallisation elimination reactions and/or decomposition reactions, is deposited onto the organic peroxycarboxylic acid, and/or is blended, in particular mixed with the peroxycarboxylic acid, in particular wherein the substance is preferably boric acid, and/or **in that** the capsule shell comprises at least one complexing agent, in particular selected from the group of quinoline and/or its salts, phosphates, alkali metal polyphosphonates, picolinic acid and dipicolinic acid, mono- or poly-phosphonic acids, in particular 1-hydroxyethylidene-1,1-diphosphonic acid (HEDP), ethylenediaminetetraacetic acid (EDTA), diethylenetriaminepenta(methylenephosphonic) acid (DTPMP), azacycloheptane diphosphonate (AHP) and/or nitrilotriacetic acid (NTA).

5. Agent according to one of claims 1 to 4, **characterised in that** the particle size of the Imidoperoxycarboxylic acid is from 10 to 3000 µm, in particular from 50 to 2500 µm, preferably from 100 to 1500 µm, and/or **in that** the particle size of the Imidoperoxycarboxylic acid used is ≤ 3000 µm, in particular ≤ 2500 µm, preferably ≤ 2250 µm, preferentially ≤ 2000 µm, particularly preferably ≤ 1500 µm, and/or **in that** the capsules loaded with at least one Imidoperoxycarboxylic acid have an average size (spherical diameter) from 10 to 4000 µm, preferably from 50 to 2000 µm, preferably 100 to 1000 µm.

6. Agent according to one of claims 1 to 5, **characterised in that** the content of the capsule shell is ≥ 5% by weight, in particular ≥ 10% by weight, preferably ≥ 20% by weight, based on the capsules, and/or **in that** the content of Imidoperoxycarboxylic acid, preferably PAP, is from 25 to 95% by weight, in particular from 30 to 90% by weight, preferably from 40 to 85% by weight, particularly preferably from 50 to 80% by weight, based on the capsules.

7. Agent according to one of claims 1 to 6, **characterised in that** at least one additional capsule shell is deposited onto the capsule shell.

8. Agent according to one of claims 1 to 7, **characterised in that** it comprises:
(i) capsules loaded with Imidoperoxycarboxylic acid in amounts of 0.1 to 30% by weight; and/or
(ii) surfactants, particularly cationic and/or anionic surfactants, preferably in amounts of 0 to 30% by weight, and/or non-ionic surfactants, preferably in amounts of 0 to 30% by weight; and/or
(iii) optionally electrolytes, particularly inorganic and/or organic salts, particularly phosphate, citrate and/or sulfate, particularly preferably sodium sulfate, preferably in amounts of 5 to 30% by weight; and/or
(iv) optionally complexing agents, particularly selected from the group of quinoline and/or its salts, alkali metal polyphosphonates, picolinic acid und dipicolinic acid, mono- or polyphosphonic acids, particularly 1-hydroxyethylidene-1,1-diphosphonic acid (HEDP), ethylenediaminetetraacetic acid (EDTA), diethylenetriaminepenta(methylenephosphonic acid) (DTPMP), azacycloheptane diphosphonate (AHP), nitrilotriacetic acid (NTA), citrate and/or short chain dicarboxylic acids, preferably in amounts of 0 to 10% by weight; and/or
(v) optionally enzymes and/or enzyme stabilisers, preferably in amounts of 0 to 10% by weight; and/or
(vi) optionally builders, in particular fatty acids and/or citric acid and/or citrate, preferably in amounts of 0 to 15% by weight; and/or
(vii) optionally fragrances, preferably in amounts of 0 to 5% by weight; and/or
(viii) optionally auxiliaries, such as defoamers, pH regulators, rheology modifiers (thickeners), solvents, colorants; and/or
(ix) optionally additional usual ingredients, such as brighteners etc.; and/or
(x) water;
wherein all the specified weights are based on the washing or cleaning agent.

9. Agent according to one of claims 1 to 8, **characterised in that** it:
- at least essentially, does not include any halide ions, particularly chloride ions, in particular wherein the amount of halide ions, particularly chloride ions, is maximum 500 ppm, preferably maximum 100 ppm, particularly preferably maximum 30 ppm; and/or
- has a pH of maximum 7, particularly a pH of 3.5 to 7, preferably from 4.0 to 6.5, particularly preferably from 4.5 to 6; and/or
- comprises at least one complexing agent, particularly selected from the group of quinoline and/or its salts, alkali metal polyphosphonates, picolinic acid und dipicolinic acid, mono- or polyphosphonic acids, particularly 1-hydroxyethylidene-1,1-diphosphonic acid (HEDP), ethylenediaminetetraacetic acid (EDTA), diethylenetriaminepenta(methylenephosphonic acid) (DTPMP), azacycloheptane diphosphonate (AHP), nitrilotriacetic acid (NTA), citrate and/or short chain dicarboxylic acids, particularly for complexing heavy metal ions; and/or
- optionally comprises at least one water-miscible solvent, in which the Imidoperoxycarboxylic acid is poorly soluble, preferably glycerine; and/or
- optionally comprises at least one catalase;
such that degradation of the Imidoperoxycarboxylic acid, preferably PAP, is at least reduced or prevented in the washing or cleaning agent.

## Revendications

1. Compositions liquides d'agent de lavage et de nettoyage, contenant des dispersions, en particulier des dispersions aqueuses, contenant des capsules chargées d'au moins un acide peroxycarboxylique organique, qui est un acide Imidoperoxycarboxylique, de préférence PAP, comprenant au moins un acide Imidoperoxycarboxylique, de préférence PAP, qui est enveloppé et/ou revêtu au moins essentiellement avec une enveloppe de capsule à base d'un sel inorganique, dans lesquelles les capsules comprennent au moins une enveloppe de capsule à base d'au moins un sel inorganique et un noyau de capsule à base d'au moins un acide Imidoperoxycarboxylique, et un sel inorganique, qui abaisse la solubilité du sel inorganique formant l'enveloppe de capsule dans la dispersion.

2. Agent selon la revendication 1, **caractérisé en ce que** l'acide Imidoperoxycarboxylique est l'acide 6-phtalimidoperoxycaproïque (acide 6-phtalimidoperoxyhexanoïque, PAP).

3. Agent selon la revendication 1 ou 2, **caractérisé en ce que** le sel inorganique est un sel non basique, de préférence un sel neutre, ou un sel en particulier faiblement acide, et/ou **en ce que** le sel inorganique est choisi dans le groupe des sels sulfate, nitrate et phosphate inorganiques, de préférence des sels sulfate inorganiques, en particulier le sel inorganique étant choisi parmi les sels de métal alcalin ou alcalino-terreux, de préférence les sels de métal alcalin, et/ou **en ce que** le sel inorganique est un sel exempt d'halogénure, en particulier un sel inorganique non basique, exempt d'halogénure, et/ou **en ce que** le sel inorganique est le sulfate de sodium.

4. Agent selon l'une quelconque des revendications 1 à 3, **caractérisé en ce qu'**une substance qui, à une température inférieure à 80°C, en particulier inférieure à 70°C, de préférence inférieure à 60°C, peut intervenir dans des réactions endothermiques, en particulier des réactions d'élimination d'eau cristalline et/ou des réactions de décomposition, est appliquée sur l'acide peroxycarboxylique organique, et/ou est mêlée, en particulier mélangée à l'acide peroxycarboxylique, en particulier la substance étant de préférence l'acide borique, et/ou **en ce que** l'enveloppe de capsule comprend au moins un agent complexant, en particulier choisi dans le groupe formé par la quinoléine et/ou ses sels, les phosphates, les polyphosphonates de métal alcalin, l'acide picolinique et l'acide dipicolinique, les acides mono- ou poly-phosphoniques, en particulier l'acide 1-hydroxyéthylidène-1,1-diphosphonique (HEDP), l'acide éthylène-diamine-tétraacétique (EDTA), l'acide diéthylènetriamine-penta(méthylènephosphonique) (DTPMP), l'azacyclo-heptane-diphosphonate (AHP) et/ou l'acide nitrilotriacétique (NTA).

5. Agent selon l'une quelconque des revendications 1 à 4, **caractérisé en ce que** la taille de particule de l'acide Imidoperoxycarboxylique est de 10 à 3 000 µm, en particulier de 50 à 2 500 µm, de préférence de 100 à 1 500 µm, et/ou **en ce que** la taille de particule de l'acide Imidoperoxycarboxylique mis en oeuvre est ≤ 3 000 µm, en particulier ≤ 2 500 µm, de préférence ≤ 2 250 µm, de préférence encore ≤ 2 000 µm, préférentiellement ≤ 1 500 µm et/ou **en ce que** les capsules chargées d'au moins un acide Imidoperoxycarboxylique présentent une taille moyenne (diamètre sphérique) de 10 à 4 000 µm, de préférence de 50 à 2 000 µm, et de préférence encore de 100 à 1 000 µm.

6. Agent selon l'une quelconque des revendications 1 à 5, **caractérisé en ce que** la teneur de l'enveloppe de capsules est ≥ 5% en poids, en particulier ≥ 10% en poids, de préférence ≥ 20% en poids, par rapport aux capsules, et/ou **en ce que** la teneur en acide Imidoperoxycarboxylique, de préférence PAP, est de 25 à 95 % en poids, en particulier de 30 à 90 % en poids, de préférence de 40 à 85 % en poids, de manière particulièrement préférée de 50 à 80 % en poids, par rapport aux capsules.

7. Agent selon l'une quelconque des revendications 1 à 6, **caractérisé en ce qu'**au moins une enveloppe de capsule supplémentaire est appliquée sur l'enveloppe de capsule.

8. Agent selon l'une quelconque des revendications 1 à 7, **caractérisé en ce qu'**il comprend:
(i) des capsules chargées d'acide Imidoperoxycarboxylique en des quantités de 0,1 à 30 % en poids; et/ou
(ii) des tensio-actifs, en particulier des tensio-actifs cationiques et/ou anioniques, de préférence en des quantités de 0 à 30 % en poids, et/ou des tensio-actifs non ioniques, de préférence en des quantités de 0 à 30 % en poids; et/ou
(iii) éventuellement des électrolytes, en particulier des sels inorganiques et/ou organiques, en particulier un phosphate, citrate et/ou sulfate, de manière particulièrement préférée le sulfate de sodium, de préférence en des quantités de 5 à 30 % en poids; et/ou
(iv) éventuellement des agents complexants, en particulier choisis dans le groupe formé par la quinoléine et/ou ses sels, les polyphosphonates de métal alcalin, l'acide picolinique et l'acide dipicolinique, les acides mono- ou poly-phosphoniques, en particulier l'acide 1-hydroxyéthylidène-1,1-diphosphonique (HEDP), l'acide éthylène-diamine-tétraacétique (EDTA), l'acide diéthylènetriamine-penta(méthylènephosphonique) (DTPMP), l'azacycloheptane-diphosphonate (AHP), l'acide nitrilotriacétique (NTA), un citrate et/ou des acides dicarboxyliques à courte chaîne, de préférence en des quantités de 0 à 10 % en poids; et/ou
(v) éventuellement des enzymes et/ou des agents stabilisants d'enzymes, de préférence en des quantités de 0 à 10 % en poids; et/ou
(vi) éventuellement des matériaux de construction, en particulier des acides gras et/ou l'acide citrique et/ou un citrate, de préférence en des quantités de 0 à 15 % en poids; et/ou
(vii) éventuellement des matières odoriférantes, de préférence en des quantités de 0 à 5 % en poids; et/ou
(viii) éventuellement des adjuvants, comme les agents anti-mousse, les régulateurs de pH, les agents modifiant la rhéologie (épaississants), les solvants, les colorants; et/ou
(ix) éventuellement d'autres ingrédients courants, comme les agents éclaircissants, etc.; et/ou
(x) de l'eau;
toutes les indications de poids étant rapportées à l'agent de lavage ou de nettoyage.

9. Agent selon l'une quelconque des revendications 1 à 8, **caractérisé en ce que**
- il ne présente au moins essentiellement pas d'ions halogénure, en particulier d'ions chlorure, en particulier la quantité d'ions halogénure, en particulier d'ions chlorure, étant au maximum de 500 ppm, de préférence au maximum de 100 ppm, de manière particulièrement préférée au maximum de 30 ppm; et/ou
- il présente une valeur de pH de 7 au maximum, en particulier une valeur de pH de 3,5 à 7, de préférence de 4,0 à 6,5, de manière particulièrement préférée de 4,5 à 6; et/ou
- il contient au moins un agent complexant, choisi en particulier dans le groupe formé par la quinoléine et/ou ses sels, les polyphosphonates de métal alcalin, l'acide picolinique et l'acide dipicolinique, les acides mono- ou poly-phosphoniques, en particulier l'acide 1-hydroxyéthylidène-1,1-diphosphonique (HEDP), l'acide éthylène-diamine-tétraacétique (EDTA), l'acide diéthylènetriamine-penta(méthylènephosphonique) (DTPMP), l'azacycloheptane-diphosphonate (AHP), l'acide nitrilotriacétique (NTA), un citrate et/ou des acides dicarboxyliques à courte chaîne, en particulier pour la complexation d'ions de métaux lourds; et/ou
- il contient éventuellement au moins un solvant miscible à l'eau ayant un faible pouvoir de dissolution pour l'acide Imidoperoxycarboxylique, de préférence le glycérol; et/ou
- il contient éventuellement au moins une catalase;
de telle manière qu'une dégradation de l'acide Imidoperoxycarboxylique, de préférence PAP, dans l'agent de lavage ou de nettoyage soit au moins diminuée ou empêchée.
